# EUROPEAN PATENT APPLICATION

(11) **EP 4 177 274 A1**
(43) Date of publication of application: **10.05.2023**
(21) Application number: 21832002.6
(22) Date of filing: 02.07.2021
(51) Int. Cl.: C07K 16/36, C12N 15/13, A61K 39/395, A61P 7/02

(54) **ANTI-FXI/FXIA ANTIBODY, ANTIGEN-BINDING FRAGMENT THEREOF, AND PHARMACEUTICAL USE THEREOF**

(30) Priority: 02.07.2020 CN 202010633951
(71) Applicant: Beijing Tuo Jie Biopharmaceutical Co. Ltd., Beijing 102206 (CN)
(72) Inventor: WANG, Lei, Beijing 102206 (CN); HE, Xugang, Beijing 102206 (CN); ZHANG, Jin, Beijing 102206 (CN); LIU, Xiao, Beijing 102206 (CN); HU, Dongmei, Beijing 102206 (CN); DU, Yanping, Beijing 102206 (CN); WU, Ran, Beijing 102206 (CN); SHEN, Chenxi, Beijing 102206 (CN); YANG, Yang, Beijing 102206 (CN); YANG, Changyong, Beijing 102206 (CN)
(74) Representative: Regimbeau
(86) International application number: PCT/CN2021/104253
(87) International publication number: WO 2022/002249

(57) **Abstract**

Provided are an anti-FXI/FXIa antibody, an antigen-binding fragment thereof, and a pharmaceutical use thereof, as well as a pharmaceutical composition comprising the anti-FXI/FXIa antibody or the antigen-binding fragment thereof, and a method for treating and preventing a disease, in particular a method for treating thrombosis or thromboembolism-related diseases or disorders.

## Description

The present application claims priority to Chinese Patent Application No. 202010633951.8 filed on July 2, 2020.

### TECHNICAL FIELD

The present application relates to an anti-FXI/FXIa antibody, an antigen-binding fragment thereof, a pharmaceutical composition comprising the anti-FXI/FXIa antibody or the antigen-binding fragment thereof, and pharmaceutical use thereof for treating or preventing a thrombosis-related disease.

### BACKGROUND

In recent years, with the aging of the global population and changes in lifestyle, the thromboembolic disease has become one of the most deadly and disabling diseases in the world. The undesirable bleeding events caused by current antithrombotic/anticoagulant drugs remain a significant problem (Katherine et al., 2015, West J Emerg Med, 16 (1): 11-17).

Available animal studies and clinical data demonstrate that inhibition of the endogenous pathway, particularly coagulation factor FXI, can reduce thrombosis without causing significant bleeding events (Felicitas Müller et al., Curr Opin Hematol. 2011 Sep; 18 (5): 349-355).

FXI is a key coagulation factor in the endogenous pathway. FXIa is the activated state of FXI. In the coagulation cascade, FXI can be activated not only by the endogenous coagulation factor FXIIa to form FXIa, but also by thrombin II, thus amplifying the coagulation cascade to form more thrombin and fibrin. In this process, too much thrombin and fibrin may be formed, resulting in thrombotic diseases. The mild bleeding phenotype of patients with human FXI deficiency (hemophilia C) reveals that the risk of bleeding is lower when FXI is suppressed. Further studies found a significant reduction in the incidence of cerebral arterial thrombosis and deep vein thrombosis in FXI-deficient patients, suggesting that inhibition of FXI is beneficial in reducing the incidence risk of cerebral arterial thrombosis and deep vein thrombosis.

The FXI gene is located on human chromosome 4 and encodes a secreted protein consisting of 607 amino acids. The FXI protein molecule contains 4 apple domains and 1 catalytic domain. FXI is present in human blood as a homodimer, and the concentration of FXI circulating in human plasma to form a non-covalent complex with HK is about 30 nM (15-45 nM). The human FXI molecule has 88%, 67% and 58% (two species, three homology data) homology to the monkey and mouse FXI molecules, respectively.

At present, there are numerous literature and clinical trials that have shown that inhibition of the activity of FXI and FXIa to which it converts is effective in reducing thrombosis without creating a visible risk of bleeding. From the clinical results disclosed so far, the anti-FXI/FXIa antibody has similar or better antithrombotic effects with lower bleeding risk and enable safer treatment of patients, as compared to the existing new oral anticoagulants (NOACs).

There remains an urgent need in the pharmaceutical field to provide an anti-FXI/FXIa antibody drug with low immunogenicity that effectively inhibits thrombosis without increasing the risk of bleeding, thereby making antithrombotic therapy safer.

### SUMMARY

The present disclosure provides an anti-FXI/FXIa antibody or an antigen-binding fragment thereof, a coding nucleic acid, vector, host cell, and pharmaceutical composition thereof, a method thereof for treating or delaying a thrombosis- or thromboembolism-related disease or disorder or complication, and use thereof for detection.

In one aspect, provided is an anti-FXI/FXIa antibody or an antigen-binding fragment thereof, comprising:
a heavy chain HCDR1 comprising a sequence set forth in X₁X₂X₃MH (SEQ ID NO: 63), wherein X₁ is selected from the group consisting of E, S, G and D, X₂ is selected from the group consisting of L, I, V and D, and X₃ is selected from the group consisting of S, F, L and Y; and/or
a heavy chain HCDR2 comprising a sequence set forth in X₄X₅DPX₆X₇GX₈TX₉YAX₁₀KFQG (SEQ ID NO: 64), wherein X₄ is selected from the group consisting of G and W, X₅ is selected from the group consisting of F and I, X₆ is selected from the group consisting of E and Q, X₇ is selected from the group consisting of D and N, X₈ is selected from the group consisting of E and D, X₉ is selected from the group consisting of I, R, V and E, and X₁₀ is selected from the group consisting of Q and S; and/or
a heavy chain HCDR3 comprising a sequence set forth in DPHRTWWRYFDWLYPRGMDV (SEQ ID NO: 9) or GNFYYFDY (SEQ ID NO: 39); and/or
a light chain LCDR1 comprising a sequence set forth in RASQTVGKNYLA (SEQ ID NO: 10) or SASSSINYMH (SEQ ID NO: 40); and/or
a light chain LCDR2 comprising a sequence set forth in X₁₁X₁₂SX₁₃X₁₄AX₁₅ (SEQ ID NO: 65), wherein X₁₁ is selected from the group consisting of G, E and D, X₁₂ is selected from the group consisting of A and T, X₁₃ is selected from the group consisting of N, V and K, X₁₄ is selected from the group consisting of R and L, and X₁₅ is selected from the group consisting of T, L and S; and/or
a light chain LCDR3 comprising a sequence set forth in X₁₇QX₁₈X₁₉X₂₀X₂₁PX₂₂T (SEQ ID NO: 66), wherein X₁₇ is selected from the group consisting of Q and H, X₁₈ is selected from the group consisting of F and R, X₁₉ is selected from the group consisting of R and S, X₂₀ is selected from the group consisting of S and F, X₂₁ is selected from the group consisting of Y and S, and X₂₂ is selected from the group consisting of Y and L.

In some embodiments, the anti-FXI/FXIa antibody or the antigen-binding fragment thereof comprises:
a heavy chain HCDR1 comprising a sequence set forth in one of SEQ ID NOs: 7, 22, 24, 26, 28 and 37;
a heavy chain HCDR2 comprising a sequence set forth in one of SEQ ID NOs: 8, 23, 25, 27 and 38;
a heavy chain HCDR3 comprising a sequence set forth in one of SEQ ID NOs: 9 and 39;
a light chain LCDR1 comprising a sequence set forth in one of SEQ ID NOs: 10 and 40;
a light chain LCDR2 comprising a sequence set forth in one of SEQ ID NOs: 11, 29 and 41; and
a light chain LCDR3 comprising a sequence set forth in one of SEQ ID NOs: 12 and 42. In some embodiments, provided is an anti-FXI/FXIa antibody or an antigen-binding fragment thereof, comprising:
   (a) heavy chain HCDR1, HCDR2 and HCDR3 comprising sequences set forth in SEQ ID NOs: 7, 8 and 9, respectively, and light chain LCDR1, LCDR2 and LCDR3 comprising sequences set forth in SEQ ID NOs: 10, 11 and 12, respectively;
   (b) heavy chain HCDR1, HCDR2 and HCDR3 comprising sequences set forth in SEQ ID NOs: 22, 23 and 9, respectively, and light chain LCDR1, LCDR2 and LCDR3 comprising sequences set forth in SEQ ID NOs: 10, 29 and 12, respectively;
   (c) heavy chain HCDR1, HCDR2 and HCDR3 comprising sequences set forth in SEQ ID NOs: 24, 25 and 9, respectively, and light chain LCDR1, LCDR2 and LCDR3 comprising sequences set forth in SEQ ID NOs: 10, 29 and 12, respectively;
   (d) heavy chain HCDR1, HCDR2 and HCDR3 comprising sequences set forth in SEQ ID NOs: 26, 27 and 9, respectively, and light chain LCDR1, LCDR2 and LCDR3 comprising sequences set forth in SEQ ID NOs: 10, 29 and 12, respectively;
   (e) heavy chain HCDR1, HCDR2 and HCDR3 comprising sequences set forth in SEQ ID NOs: 28, 25 and 9, respectively, and light chain LCDR1, LCDR2 and LCDR3 comprising sequences set forth in SEQ ID NOs: 10, 29 and 12, respectively;
   (f) heavy chain HCDR1, HCDR2 and HCDR3 comprising sequences set forth in SEQ ID NOs: 37, 38 and 39, respectively, and light chain LCDR1, LCDR2 and LCDR3 comprising sequences set forth in SEQ ID NOs: 40, 41 and 42, respectively;
   (g) heavy chain HCDRs having 0-10 amino acid mutations as compared to the HCDRs of any one of heavy chains of (a)-(f), and light chain CDRs having 0-10 amino acid mutations as compared to the LCDRs of any one of light chains of (a)-(f).

In some embodiments, when the anti-FXI/FXIa antibody or the antigen-binding fragment thereof comprises CDR sequences of (a), (b), (c), (d), (e) or related (g) scheme, it selectively binds to FXIa but not FXI.

In some other embodiments, when the anti-FXI/FXIa antibody or the antigen-binding fragment thereof comprises CDR sequences of (f) or related (g) scheme, it binds to FXI, as well as to FXIa; in some specific embodiments, it binds to FXIa but does not affect the activity of FXIa.

In some embodiments, provided is an anti-FXI/FXIa antibody or an antigen-binding fragment thereof, which is a murine antibody, a chimeric antibody, a humanized antibody, or a human antibody, or a fragment thereof; in some specific embodiments, which is a humanized antibody or a human antibody or a fragment thereof. It may be a full-length antibody or a fragment thereof.

In some embodiments, the anti-FXI/FXIa antibody or the antigen-binding fragment thereof is a humanized antibody or a fragment thereof, wherein the humanized antibody may have a light chain template of IGKV3-11*01 and a heavy chain template of IGHV1-69-2*01.

In some embodiments, the humanization process also comprises back mutations to VH and VL. In some specific embodiments, for example, the VH has a back mutation of any one or any combination of Y27F, T28N, F29I, T30K, A93L, R94Y, E73T, R66K, V67A, T75A and T76N. In some specific embodiments, the VL has a back mutation of any one or any combination of R45K, L46R, L47W, I58V and F71Y

In some specific embodiments, the humanized antibody or the fragment thereof described above comprise heavy chain HCDR1, HCDR2 and HCDR3 comprising sequences set forth in SEQ ID NOs: 37, 38 and 39, respectively, and light chain LCDR1, LCDR2 and LCDR3 comprising sequences set forth in SEQ ID NOs: 40, 41 and 42, respectively.

In some embodiments, the anti-FXI/FXIa antibody or the antigen-binding fragment thereof comprises:
a VH set forth in one of SEQ ID NOs: 5, 17-20, 30-33, 35, 43, 45-49, and 53-58 or a VH having at least 80% identity thereto, and/or
a VL set forth in one of SEQ ID NOs: 6, 21, 34, 36, 44 and 50-52 or a VL having at least 80% identity thereto.

In some specific embodiments, the anti-FXI/FXIa antibody or the antigen-binding fragment thereof comprises:
(h) a VH set forth in SEQ ID NO: 5 or a VH having at least 80% identity thereto, and a VL set forth in SEQ ID NO: 6 or a VL having at least 80% identity thereto;
(i) a VH set forth in SEQ ID NO: 17 or a VH having at least 80% identity thereto, and a VL set forth in SEQ ID NO: 21 or a VL having at least 80% identity thereto;
(j) a VH set forth in SEQ ID NO: 18 or a VH having at least 80% identity thereto, and a VL set forth in SEQ ID NO: 21 or a VL having at least 80% identity thereto;
(k) a VH set forth in SEQ ID NO: 19 or a VH having at least 80% identity thereto, and a VL set forth in SEQ ID NO: 21 or a VL having at least 80% identity thereto;
(l) a VH set forth in SEQ ID NO: 20 or a VH having at least 80% identity thereto, and a VL set forth in SEQ ID NO: 21 or a VL having at least 80% identity thereto;
(m) a VH set forth in SEQ ID NO: 30 or a VH having at least 80% identity thereto, and a VL set forth in SEQ ID NO: 34 or a VL having at least 80% identity thereto;
(n) a VH set forth in SEQ ID NO: 31 or a VH having at least 80% identity thereto, and a VL set forth in SEQ ID NO: 34 or a VL having at least 80% identity thereto;
(o) a VH set forth in SEQ ID NO: 32 or a VH having at least 80% identity thereto, and a VL set forth in SEQ ID NO: 34 or a VL having at least 80% identity thereto;
(p) a VH set forth in SEQ ID NO: 35 or a VH having at least 80% identity thereto, and a VL set forth in SEQ ID NO: 36 or a VL having at least 80% identity thereto;
(q) a VH set forth in SEQ ID NO: 43 or a VH having at least 80% identity thereto, and a VL set forth in SEQ ID NO: 44 or a VL having at least 80% identity thereto;
(r) a VH set forth in SEQ ID NO: 45 or a VH having at least 80% identity thereto, and a VL set forth in SEQ ID NO: 51 or a VL having at least 80% identity thereto;
(s) a VH set forth in SEQ ID NO: 49 or a VH having at least 80% identity thereto, and a VL set forth in SEQ ID NO: 51 or a VL having at least 80% identity thereto;
(t) a VH set forth in SEQ ID NO: 58 or a VH having at least 80% identity thereto, and a VL set forth in SEQ ID NO: 51 or a VL having at least 80% identity thereto.

In some embodiments, the anti-FXI/FXIa antibody or the antigen-binding fragment thereof has a VH linked to human or mouse CH1 and a VL linked to human or mouse CL or Cx. The human CH is set forth in SEQ ID NO: 13 or 59, and the Cκ is for example set forth in SEQ ID NO: 14.

In some embodiments, the anti-FXI/FXIa antibody or the antigen-binding fragment thereof is a murine antibody or a fragment thereof. The light chain variable region comprises a light chain FR region and/or a light chain constant region of a murine κ chain or λ chain or a variant thereof. In some specific embodiments, the murine anti-FXI/FXIa antibody or the antigen-binding fragment thereof comprises a heavy chain FR region and/or a heavy chain constant region of murine IgG1, IgG2, IgG3 or IgG4, or a variant thereof.

In some embodiments, the anti-FXI/FXIa antibody or the antigen-binding fragment thereof is a chimeric antibody or a fragment thereof. It comprises a light chain FR region and/or a light chain constant region of a human κ or λ chain or a variant thereof and/or a heavy chain FR region and/or a heavy chain constant region of human IgG1, IgG2, IgG3 or IgG4, or a variant thereof.

In some embodiments, the anti-FXI/FXIa antibody or the antigen-binding fragment thereof comprises a constant region Fc, e.g., the Fc of IgG1, IgG2, IgG3, IgG4 or IgG4P (i.e., the S241P mutant of IgG4). The sequence of the Fc of IgG1 is for example set forth in SEQ ID NO: 67, and the sequence of the IgG4P Fc (i.e., IgG4Fc comprising S241P) is for example set forth in SEQ ID NO: 60.

In some embodiments, the anti-FXI/FXIa antibody or the antigen-binding fragment thereof has a heavy chain set forth in SEQ ID NO: 15 or a heavy chain having at least 80% identity hereto, and a light chain set forth in SEQ ID NO: 16 or a light chain having at least 80% identity thereto; or
a heavy chain set forth in SEQ ID NO: 61 or a heavy chain having at least 80% identity thereto, and a light chain set forth in SEQ ID NO: 62 or a light chain having at least 80% identity thereto.

In some embodiments, the antigen-binding fragment of the anti-FXI/FXIa antibody is a Fab, an Fv, an sFv, a Fab', an F(ab')₂, a linear antibody, a single-chain antibody, an scFv, an sdAb, an sdFv, a nanobody, a peptibody, a domain antibody, and a multispecific antibody (bispecific antibody, diabody, triabody, and tetrabody, tandem di-scFv, tandem tri-scFv), for example, specifically, an scFv, Fv, Fab or Fab' fragment.

In some embodiments, provided is an anti-FXI/FXIa antibody or an antigen-binding fragment thereof, which binds to the same epitope as the anti-FXI/FXIa antibody or the antigen-binding fragment thereof described above.

In some other embodiments, provided is an anti-FXI/FXIa antibody or an antigen-binding fragment thereof, which cross-blocks the binding of the anti-FXI/FXIa antibody or the antigen-binding fragment thereof described above to human FXI/FXIa.

In some other embodiments, provided is an anti-FXI/FXIa antibody or an antigen-binding fragment thereof, the binding of which to human FXI/FXIa is cross-blocked by the anti-FXI/FXIa antibody or the antigen-binding fragment thereof described above.

In some embodiments, provided is an anti-FXI/FXIa antibody or an antigen-binding fragment thereof, which comprises a heavy and/or light chain having at least 80% identity to the heavy and/or light chain of the anti-FXI/FXIa antibody or the antigen-binding fragment thereof described above.

In the context of the present disclosure, "at least 80%" encompasses 80% or more, e.g., at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%.

In some embodiments, provided is a variant of an anti-FXI/FXIa antibody or an antigen-binding fragment thereof, which comprises 0, 1, 2, 3, 4, 5, 6, 7, 8, 9 or 10 amino acid changes in the heavy chain variable region and/or the light chain variable region of the anti-FXI/FXIa antibody or the antigen-binding fragment thereof described above. The amino acid change may be a conservative substitution of an amino acid residue in the variable region.

In some embodiments, the anti-FXI/FXIa antibody or the antigen-binding fragment thereof described above may have any one or more of the following properties: preventing activation of the intrinsic or common coagulation pathway;
blocking binding of FXI and/or FXIa to a member of the coagulation pathway (preferably, the member is selected from one or more of factor IX, factor XIIa and thrombin);
blocking binding of one or more of FIX, FXI and FXIa to a platelet receptor;
having a KD for binding to a human FXI and/or FXIa protein of ≤ 10⁻⁹;
preventing the FXI/FXIa catalytic domain from presenting in an active conformation when binding to FXI/FXIa; and
having the ability to be used for subcutaneous administration or intravenous administration.

The affinity described above is measured, for example, by BIACORE^{™}.

In another aspect, the present disclosure provides a polynucleotide encoding any one of the anti-FXI/FXIa antibodies or the antigen-binding fragments thereof, which may be DNA or RNA.

In yet another aspect, the present disclosure provides an expression vector comprising the polynucleotide described above, which is selected from the group consisting of a eukaryotic expression vector, a prokaryotic expression vector and a viral vector.

In yet another aspect, the present disclosure provides a host cell transformed with the expression vector described above, which may be a eukaryotic cell or a prokaryotic cell. In some embodiments, the host cell is selected from the group consisting of bacteria, yeast and a mammalian cell. In some specific embodiments, the host cell is selected from the group consisting of *Escherichia coli, Pichia pastoris,* a Chinese hamster ovary (CHO) cell and a human embryonic kidney (HEK) 293 cell.

In yet another aspect, the present disclosure provides a method for preparing an anti-FXI/FXIa antibody or an antigen-binding fragment thereof, which comprises: expressing the antibody or the antigen-binding fragment thereof in the host cell described above, and isolating the antibody or the antigen-binding fragment thereof from the host cell.

In yet another aspect, the present disclosure provides a composition, e.g., a pharmaceutical composition, which comprises: a pharmaceutically acceptable excipient, diluent or carrier; and a therapeutically or prophylactically effective amount of the anti-FXI/FXIa antibody or the antigen-binding fragment thereof described above. In some specific embodiments, the pharmaceutical composition may contain 0.01 wt% to 99 wt% of the anti-FXI/FXIa antibody or the antigen-binding fragment thereof in a unit dose, or the pharmaceutical composition may contain 0.1-2000 mg, in some specific embodiments, 1-1000 mg of the anti-FXI/FXIa antibody or the antigen-binding fragment thereof in a unit dose.

In some specific embodiments, the pharmaceutical composition described above is in a dosage form for subcutaneous administration or intravenous administration.

In some embodiments, provided is a pharmaceutical composition for subcutaneous administration, which comprises a therapeutically or prophylactically effective amount of the anti-FXI/FXIa antibody or the antigen-binding fragment thereof of the present disclosure described above. For example, the antibody comprises a heavy chain variable region comprising a sequence set forth in SEQ ID NO: 58 and a light chain variable region comprising a sequence set forth in SEQ ID NO: 51; for example, the antibody comprises a full length heavy chain comprising a sequence set forth in SEQ ID NO: 61 and a full length light chain comprising a sequence set forth in SEQ ID NO: 62.

In yet another aspect, the present disclosure provides use of any one or any combination selected from the group consisting of the following in the preparation of a medicament: the anti-FXI/FXIa antibody or the antigen-binding fragment thereof according to the present disclosure, and the pharmaceutical composition according to the present disclosure.

In some embodiments, the medicament is for use in treating or preventing a thrombosis- or thromboembolism-related disease or disorder, e.g., ischemic stroke and/or deep vein thrombosis related to atrial fibrillation.

The present disclosure provides a method for treating or preventing a thrombosis- or thromboembolism-related disease or disorder (or a complication thereof), or delaying progression of a thrombosis- or thromboembolism-related disease or disorder (or a complication thereof), which comprises administering to a subject the anti-FXI/FXIa antibody or the antigen-binding fragment thereof according to the present disclosure, or the pharmaceutical composition according to the present disclosure in an effective amount for treating or delaying the disease.

The anti-FXI/FXIa antibody or the antigen-binding fragment thereof, and the pharmaceutical composition of the present disclosure may be applied to a disease or disorder related to clot formation, thrombosis, or thromboembolism.

The thrombosis- or thromboembolism-related disease or disorder includes, but is not limited to: cardiac coronary artery disease (e.g., acute coronary syndrome (ACS)), ST-elevation myocardial infarction (STEMI) and non-ST-elevation myocardial infarction (non-STEMI), stable angina pectoris, unstable angina pectoris, reocclusion and restenosis after coronary interventions (such as angioplasty, stenting or aortocoronary bypass), peripheral arterial occlusive disease, pulmonary embolism, venous thromboembolism, venous thrombosis (especially in the deep veins of the lower extremities and renal veins), transient ischemic attack, thrombotic stroke and thromboembolic stroke, pulmonary disease or pulmonary hypertension caused by chronic thromboembolic pulmonary hypertension (CTEPH).

Stimulation of the coagulation system can occur through a variety of causes or associated disorders. In the case of surgical intervention, immobility, bed rest, infection, inflammation or cancer, cancer treatment or the like, the coagulation system can be highly activated, and there may be thrombotic complications, in particular venous thrombosis. Thus, the anti-FXI/FXIa antibody or the antigen-binding fragment thereof, and the pharmaceutical composition of the present disclosure may be used for preventing thrombosis in the case of surgical intervention, particularly for patients with cancer or patients undergoing plastic surgery (such as hip or knee arthroplasty). Thus, the anti-FXI/FXIa antibody or the antigen-binding fragment thereof, and the pharmaceutical composition of the present disclosure are also used for preventing thrombosis in patients with an activated coagulation system, e.g., in the case of the stimulation.

The anti-FXI/FXIa antibody or the antigen-binding fragments thereof, and the pharmaceutical composition of the present disclosure are used for treating and/or preventing cardiogenic thromboembolism (e.g., cerebral arterial ischemia, stroke, and systemic and local thromboembolism) in patients with acute, intermittent or perpetual arrhythmia (e.g., atrial fibrillation), or undergoing cardioversion, or suffering from heart valve disease, or with prosthetic heart valves.

The anti-FXI/FXIa antibody or the antigen-binding fragment thereof, and the pharmaceutical composition of the present disclosure are used for treating and/or preventing disseminated intravascular coagulation (DIC). The disease may occur particularly in association with sepsis or due to surgical intervention, neoplastic disease, burns or other injuries, and may cause serious organ damage through microthrombosis. The anti-FXI/FXIa antibody or the antigen-binding fragment thereof, and the pharmaceutical composition of the present disclosure are used for treating and/or preventing thromboembolic complications. For example, the complications may occur in microangiopathic hemolytic anemia and, in the case of extracorporeal circulation, may result from the contact of the blood with exogenous surfaces (such as hemodialysis, ECMO ("extracorporeal membrane oxygenation"), LVAD ("left ventricular assist device"), and similar methods).

The anti-FXI/FXIa antibody or the antigen-binding fragment thereof, and the pharmaceutical composition of the present disclosure are used for treating and/or preventing diseases involving microclot formation or fibrin deposition in cerebral blood vessels, which may lead to dementia, such as vascular dementia or Alzheimer's disease. The anti-FXI/FXIa antibody or the antigen-binding fragment thereof, and the pharmaceutical composition of the present disclosure are used for preventing and/or treating thrombotic and/or thromboembolic complications, e.g., venous thromboembolism, in cancer patients, particularly those patients who have undergone major surgical intervention or chemotherapy or radiotherapy.

The anti-FXI/FXIa antibody or the antigen-binding fragments thereof, and the pharmaceutical composition of the present disclosure are used for treating and/or preventing disseminated intravascular coagulation in the context of the following diseases, including but not limited to: infectious disease and/or systemic inflammatory response syndrome (SIRS), septic organ dysfunction, septic organ failure and multiple organ failure, acute respiratory distress syndrome (ARDS), acute lung injury (ALI), septic stroke, and/or septic organ failure. In the case of infection, there may be a general activation of the coagulation system (disseminated intravascular coagulation, also known as "DIC"), with microthrombosis in multiple organs and secondary bleeding complications. There may be endothelial damage, with increased vascular permeability and diffusion (e.g., exosmosis) of body fluids and proteins to the space. As the infection progresses, there may be organ failure (e.g., renal failure, hepatic failure, respiratory failure, central nervous function defect, and cardiovascular failure) or multiple organ failure. In the case of DIC, there is massive activation of the coagulation system on the surface of damaged endothelial cells, foreign bodies or crosslinked extravascular tissue. Thus, coagulation occurs in the small blood vessels of multiple organs with ischemia and subsequent organ dysfunction. The secondary effect is the consumption of coagulation factors (e.g., factor X, prothrombin and fibrinogen) and platelets, which reduce the coagulability of the blood and may lead to severe bleeding.

The anti-FXI/FXIa antibody or the antigen-binding fragment thereof, and the pharmaceutical composition of the present disclosure are used for treating and/or preventing a thrombotic or thromboembolic disease and/or an inflammatory disease and/or a disease with increased vascular permeability in a patient who has a gene mutation that leads to an increase in enzyme activity or an increase in the level of proenzyme, as determined by relevant experiments/measurements of enzyme activity or concentration of proenzyme.

The present disclosure provides use of the anti-FXI/FXIa antibody or the antigen-binding fragment thereof, and the pharmaceutical composition of the present disclosure for the preparation of a medicament for treating and/or preventing a disease, particularly the disease described above, for example for the preparation of a medicament for treating and/or preventing a thrombotic or thromboembolic disease. The present disclosure provides a method of the anti-FXI/FXIa antibody or the antigen-binding fragment thereof, and the pharmaceutical composition of the present disclosure for treating and/or preventing the disease described above.

In yet another aspect, the present disclosure provides a composition for detecting FXI/FXIa, wherein the composition comprises an anti-FXI/FXIa antibody or an antigen-binding fragment thereof. The present disclosure also provides a method, system or device for detecting FXI/FXIa *in vivo* or *in vitro,* which comprises processing an anti-FXI/FXIa antibody.

In some embodiments, the method, system or device for detecting FXI/FXIa *in vitro* may, for example, comprise:
(1) making a sample to be tested in contact with an antibody or an antigen-binding fragment thereof that binds to FXI/FXIa;
(2) detecting a complex formed between the antibody or the antigen-binding fragment thereof that binds to FXI/FXIa and the sample to be tested; and/or
(3) making a reference sample (e.g., a control sample) in contact with the antibody; and
(4) determining the extent of formation of the complex between the antibody and the sample to be tested by comparing with the reference sample.

For example, a change (e.g., a statistically significant change) in the formation of the complex in the sample to be tested or subject as compared to that in the control sample or subject indicates the presence of FXI/FXIa in the sample to be tested.

In some other embodiments, the method, system or device for detecting FXI/FXIa *in vivo* may comprise:
(1) administering to a subject an antibody or an antigen-binding fragment thereof that binds to FXI/FXIa; and
(2) detecting a complex formed between the antibody or the antigen-binding fragment thereof that binds to FXI/FXIa and a substance to be tested.

The detection may include determining the location or time at which the complex is formed. The antibody that binds to FXI/FXIa may be directly or indirectly labeled with a detectable substance to facilitate the detection of bound or unbound antibodies. Suitable detectable substances include various enzymes, prosthetic groups, fluorescent materials, luminescent materials and radioactive materials. The formation of a complex between an antibody or an antigen-binding fragment thereof that binds to FXI/FXIa and FXI/FXIa can be detected by determining or visualizing the antibody that binds to or does not bind to FXI/FXIa. Conventional detection assays may be used, such as enzyme-linked immunosorbent assay (ELISA), radioimmunoassay (RIA) or tissue immunohistochemistry.

In some embodiments, the sample is analyzed for the presence of FXI/FXIa by a competitive immunoassay that uses a marker labeled with a detectable substance and an unlabeled antibody that binds to FXI/FXIa.

In some embodiments, the antibody or the fragment thereof of the present disclosure may be labeled with a fluorophore and a chromophore for detection purposes.

In some embodiments, also provided is a kit, which comprises an anti-FXI/FXIa antibody or an antigen-binding fragment thereof; and optionally, may comprise instructions for diagnostic use. The kit may also comprise at least one additional reagent, such as a label or an additional diagnostic agent. For *in vivo* use, the antibody may be formulated into a pharmaceutical composition.

### BRIEF DESCRIPTION OF THE DRAWINGS

FIGs. 1A to 1B: assay on the binding of FXI/FXIa antibodies to a human FXI/FXIa protein by SPR. FIG. 1A shows the analysis results of the binding of the 3882 molecule to FXI by SPR; and FIG. 1B shows the analysis results of the binding of the 1209 molecule to FXIa by SPR.
FIGs. 2A to 2B: assay on the inhibition of FXIa enzyme activity by the FXI/FXIa antibodies *in vitro.* FIG. 2A shows the assay results of inhibition of FXIa enzyme activity by anti-FXI/FXIa antibodies *in vitro;* and FIG. 2B shows the assay results of FXIIa-mediated inhibition of FXI activating enzyme activity by anti-FXI/FXIa antibodies *in vitro.*
FIGs. 3A to 3B: assay on the aPTT and PT anticoagulant activity in human blood. FIGs. 3A and 3B show the assay results of aPTT and PT of anti-FXI/FXIa antibodies in human blood, respectively.
FIGs. 4A to 4B: assay on the aPTT and PT anticoagulant activity in monkey blood.

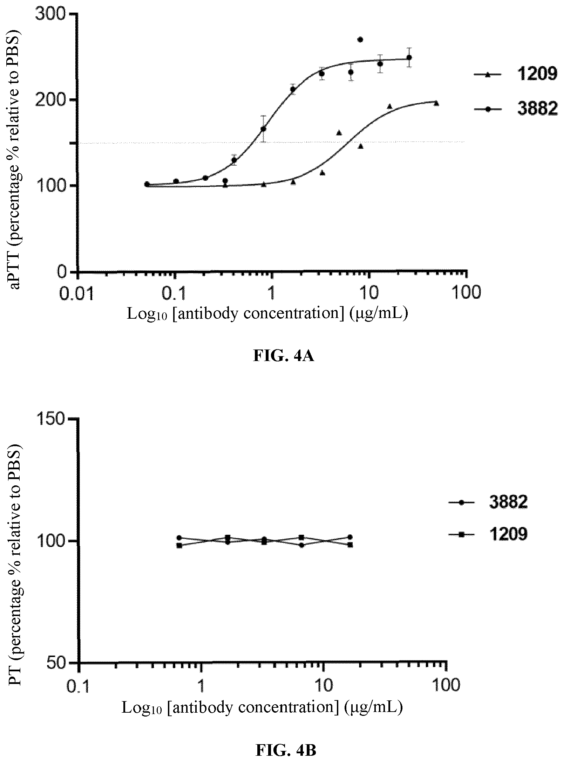

FIGs. 4A and 4B show the assay results of aPTT and PT of anti-FXI/FXIa antibodies in monkey blood, respectively.

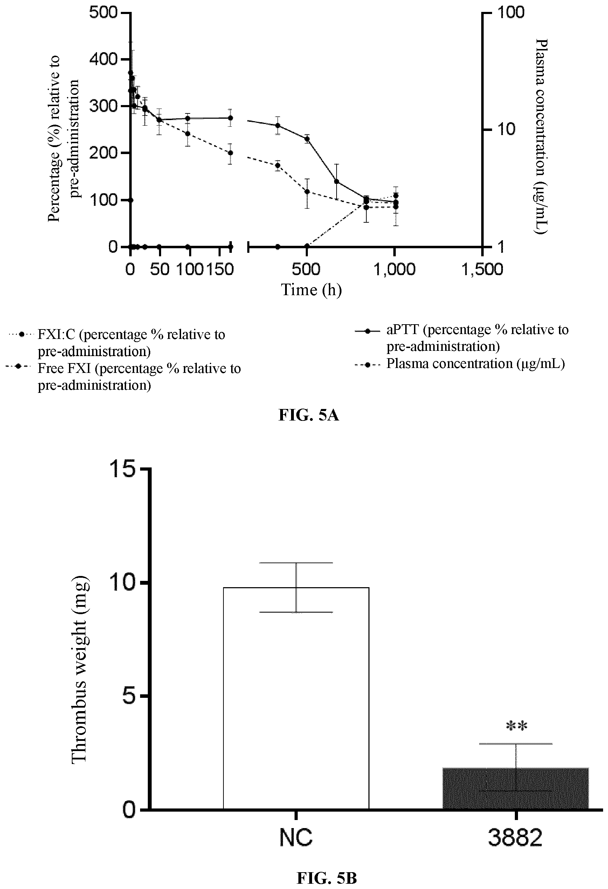

FIGs. 5A to 5D: animal experiment on FXI/FXIa antibodies in cynomolgus monkeys.

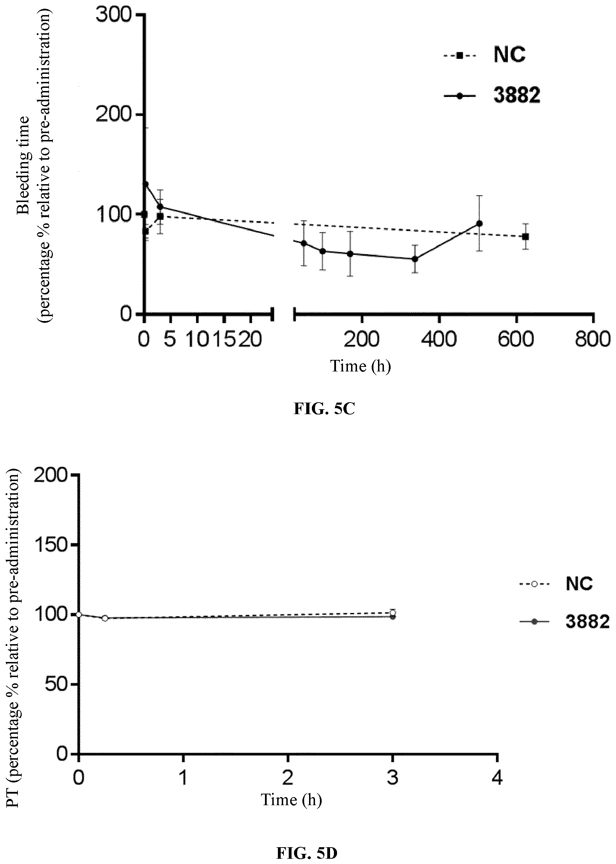

FIG. 5A shows curves of change in aPTT, plasma drug concentration, FXI:C % and free FXI of antibody 3882 in cynomolgus monkeys; FIG. 5B shows the inhibition effect of 3882 on thrombosis in cynomolgus monkeys; and FIGs. 5C and 5D show safety tests of 3882 in cynomolgus monkeys, with bleeding time determination and PT assay results shown, respectively.

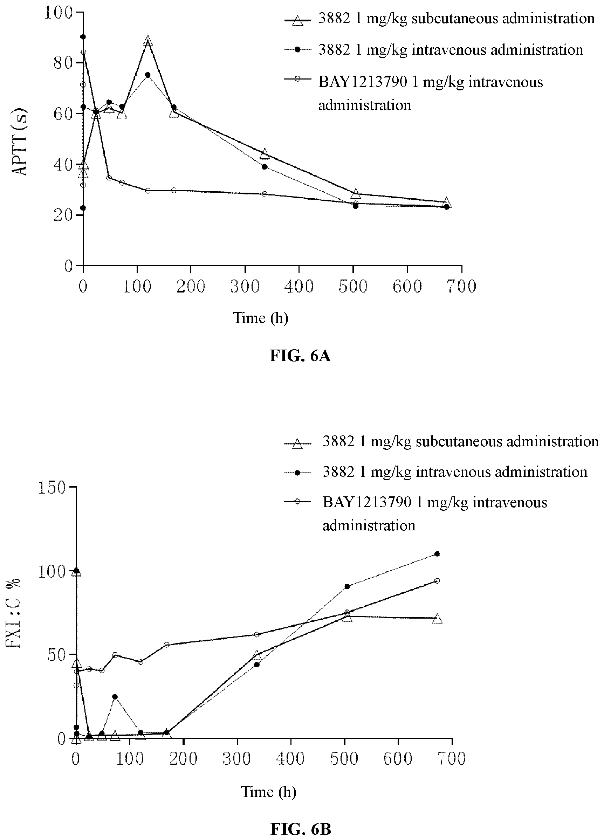

FIGs. 6A to 6B: assay results of pharmacodynamics (PD) of FXI/FXIa antibodies in cynomolgus monkeys. FIG. 6A shows assay results of APTT(s); and FIG. 6B shows assay results of FXI:C (%), wherein 3882 (1 mg/kg) was administered intravenously and subcutaneously, and the control BAY1213790 (1 mg/kg) was administered intravenously.

### DETAILED DESCRIPTION

### Terms

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs. The three-letter and single-letter codes for amino acids used in the present disclosure are described as in J. Biol. Chem, 243, p3558 (1968).

"Factor XI", also referred to herein as "coagulation factor XI", "FXI" or "fXI", is a double-stranded glycoprotein having a molecular weight of about 160 kilodaltons (kD). Both chains may be identical polypeptides having a molecular weight of about 80,000 daltons; and are linked by disulfide bonds. FXI contains 4 "apple domains" (A1-A4 from the N-terminus, the heavy chain) and the C-terminal catalytic domain (the light chain). Without wishing to be bound by a particular theory, it is believed that the four apple domains contain binding sites for other proteins, such as A1 for thrombin; A2 for HK; A3 for factor IX (FIX), GPIb and heparin; and A4 for FXIIa. FXI can be converted to its active form, i.e., factor XIa (FXIa), by factor XIIa (FXIIa). The serine protease FXIa converts factor IX to IXa, which in turn activates factor X (Xa). Xa can then mediate the activation of coagulation factor II/thrombin.

FXI and FXIa should be understood in the broadest sense within the scope of the present disclosure. The term encompasses natural forms and naturally occurring variants of FXI and FXIa in nature, also including artificially expressed forms. FXI (or FXIa) encompasses the category of intact proteins and their epitopes in cases where antigen-antibody interactions are involved, when it is not specifically stated in the context.

"Antibody" is used in the broadest sense and encompasses a variety of antibody structures, including but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding portions) so long as they exhibit the desired antigen-binding activity. An antibody may refer to an immunoglobulin, which is of a tetrapeptide chain structure formed by two heavy chains and two light chains linked by interchain disulfide bonds. The heavy chain constant regions of an immunoglobulin differ in their amino acid composition and arrangement, and thus in their antigenicity. Accordingly, immunoglobulins can be divided into five classes, or isotypes of immunoglobulins, namely IgM, IgD, IgG, IgA and IgE, with their corresponding heavy chains being µ chain, δ chain, γ chain, α chain and ε chain, respectively. Ig of the same class can be divided into different subclasses according to differences in the amino acid composition of the hinge regions and the number and positions of disulfide bonds of the heavy chains; for example, IgG may be divided into IgG1, IgG2, IgG3 and IgG4. Light chains are classified into κ or λ chains by the differences in the constant regions. Each of the five classes of Ig may have a κ chain or λ chain. In the heavy and light chains of antibody, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity determining regions (CDRs). Each of the light chain variable regions (VLs) and the heavy chain variable regions (VHs) consists of 3 CDR regions and 4 FR regions arranged from the amino terminus to the carboxyl terminus in the following order: FR1, CDR1, FR2, CDR2, FR3, CDR3, and FR4. The 3 CDR regions of the light chain refer to LCDR1, LCDR2, and LCDR3; and the 3 CDR regions of the heavy chain refer to HCDR1, HCDR2, and HCDR3.

For determination or definition of "CDRs", the deterministic depiction of CDRs and identification of residues comprising antigen-binding sites of the antibody can be accomplished by resolving the structure of the antibody and/or resolving the structure of the antibody-ligand complex. This can be accomplished by any of a variety of techniques known to those skilled in the art, such as X-ray crystallography. A variety of analysis methods can be used to identify CDRs, including but not limited to Kabat numbering scheme, Chothia numbering scheme, AbM numbering scheme, IMGT numbering scheme, contact definition, and conformational definition.

The Kabat numbering scheme is a standard for numbering residues in antibodies and is generally used to identify CDR regions (see, e.g., Johnson & Wu, 2000, Nucleic Acids Res., 28: 214-218).

The Chothia numbering scheme is similar to the Kabat numbering scheme, except that it takes into account the location of certain structural loop regions (see, e.g., Chothia et al., 1986, J. Mol. Biol., 196: 901-917; Chothia et al., 1989, Nature, 342: 877-883).

The AbM numbering scheme adopts a computer program integration suite for modeling antibody structures manufactured by Oxford Molecular Group (see, e.g., Martin et al., 1989, Proc Natl Acad Sci (USA), 86: 9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3: 194-198).

The contact definition is based on the analysis of the available complex crystal structures (see, e.g., MacCallum et al., 1996, J. Mol. Biol., 5: 732-745). In the conformational definition, the positions of the CDRs can be identified as residues that contribute enthalpy to the antigen binding (see, e.g., Makabe et al., 2008, Journal of Biological Chemistry, 283: 1156-1166).

Other CDR boundary definitions may not strictly follow one of the above methods, but still overlap with at least a portion of the Kabat CDRs, although they may be shortened or lengthened based on predictions or experimental results that a particular residue or group of residues does not significantly affect the antigen binding. As used herein, a CDR may refer to a CDR defined by any method known in the art, including combinations of methods.

The CDR amino acid residues of the VL and VH regions of the antibody or the antigen-binding fragment of the present disclosure correspond with known Kabat numbering scheme in terms of number and positions. When the Kabat numbering scheme is not adopted, the skilled person can determine corresponding equivalent sites in different numbering schemes, as compared to amino acid sites in CDRs of the present disclosure. For another example, the skilled person can also determine such equivalent sites by alignment analysis of the amino acid sequences.

"CL", "CL region" and "CL domain" are used interchangeably herein to refer to a light chain constant region.

"CH", "CH region" and "CH domain" are used interchangeably herein to refer to the heavy chain constant region and include the "CH1", "CH2" and "CH3" regions or domains.

"Human antibody" or "recombinant human antibody" includes human antibodies prepared, expressed, created, or isolated by recombinant methods, involving techniques and methods well known in the art, such as:
(1) antibodies isolated from transgenic human immunoglobulin genes, transchromosomal animals (e.g., mice), or hybridomas prepared therefrom;
(2) antibodies isolated from host cells that have been transformed to express the antibodies, such as transfectomas;
(3) antibodies isolated from a recombinant combinatorial human antibody library; and
(4) antibodies prepared, expressed, created or isolated by a method such as splicing human immunoglobulin gene sequences to other DNA sequences.

Such recombinant human antibodies comprise variable and constant regions that utilize specific human germline immunoglobulin sequences encoded by germline genes, and also include subsequent rearrangements and mutations which occur, for example, during antibody maturation.

The term "murine antibody" in the present disclosure refers to a monoclonal antibody against human FXI/FXIa or an epitope thereof prepared according to the knowledge and skill in the art. During the preparation, a test subject is injected with an FXI/FXIa antigen, and then hybridomas expressing antibodies with desired sequence or functional properties are isolated. In a specific embodiment of the present disclosure, the murine anti-human FXI/FXIa antibody or the antigen-binding fragment thereof may further comprise a light chain constant region of a murine κ or λ chain or a variant thereof, or further comprise a heavy chain constant region of a murine IgG1, IgG2, IgG3 or IgG4 or a variant thereof.

The term "human antibody" includes antibodies having variable and constant regions derived from human germline immunoglobulin sequences. The human antibody of the present disclosure may include amino acid residues not encoded by human germline immunoglobulin sequences (e.g., mutations introduced by random or site-specific mutagenesis *in vitro* or by somatic mutations *in vivo*)*.* However, the term "human antibody" does not include humanized antibodies.

The term "humanized antibody", also known as a CDR-grafted antibody, refers to an antibody produced by grafting CDR sequences of non-human species into the framework of variable regions of a human antibody. Such an antibody can overcome the strong immune response induced by the chimeric antibody because of carrying a large amount of heterologous protein components. To avoid the decrease in activity caused by the decrease in immunogenicity, the variable regions of a human antibody can be subjected to minimum reverse mutation to maintain activity.

The term "chimeric antibody" refers to an antibody obtained by fusing a variable region of an antibody of a first species to a constant region of a second species, which can reduce an immune response induced by the antibody of the first species. As an example, the chimeric antibody is established by firstly establishing hybridoma secreting murine specific monoclonal antibody, then cloning a variable region gene from the mouse hybridoma cells, cloning a constant region gene of human antibody as required, linking the mouse variable region gene and the human constant region gene into a chimeric gene, inserting the chimeric gene into a human vector, and finally expressing chimeric antibody molecules in a eukaryotic industrial system or prokaryotic industrial system. The constant region of the human antibody may be selected from the group consisting of the heavy chain constant regions of human IgG1, IgG2, IgG3 and IgG4 or variants thereof, preferably comprising heavy chain constant regions of human IgG2 or IgG4, or IgG1 mutated at amino acids without ADCC (antibody-dependent cell-mediated cytotoxicity) toxicity.

The term "antigen-binding fragment" includes a single-chain antibody (i.e., heavy and light chains); a Fab, a modified Fab, a Fab', a modified Fab', an F(ab')₂, an Fv, an Fab-Fv, an Fab-dsFv, a single domain antibody (e.g., VH or VL or VHH), an scFv, a bivalent or trivalent or tetravalent antibody, a Bis-scFv, a diabody, a tribody, a triabody, a tetrabody and an epitope-binding fragment of any one of the above (see, e.g., Holliger and Hudson, 2005, Nature Biotech. 23 (9): 1126-1136; Adair and Lawson, 2005, Drug Design Reviews-Online 2 (3), 209-217). Methods for producing and preparing such antibody fragments are well known in the art (see, e.g., Verma et al., 1998, Journal of Immunological Methods, 216, 165-181). Fab-Fv was first disclosed in WO2009/040562, and its disulfide-stabilized form Fab-dsFv was first disclosed in WO2010/035012. The antigen-binding fragment of the present disclosure also include Fab and Fab' fragments described in WO2005/003169, WO2005/003170 and WO2005/003171. Multivalent antibodies may include multispecific, e.g., bispecific or monospecific antibodies (see, e.g., WO92/22583 and WO05/113605).

"Variant" refers to a polypeptide comprising at least one amino acid modification (such as a substitution, deletion or insertion) as compared to the "parent" amino acid sequence, so long as the variant is still capable of binding to FXI/FXIa, particularly human FXI/FXIa set forth in SEQ ID NO: 1. Preferably, the variant exhibits similar or even improved properties as compared to antibodies 0012, 1209, 1267, 3807, 3871 and 3882 in the examples of the present disclosure. Variants of the binding molecules (e.g., the antibody or the antigen-binding fragment thereof) of the present disclosure are generally prepared by introducing appropriate nucleotide changes into the nucleic acid encoding the antibody or the antibody fragment or by peptide synthesis. Generally, the amino acid modification described above may be introduced into the variable or constant regions. For example, as compared to the anti-FXI/FXIa antibody in the examples of the present disclosure, amino acid modifications may be introduced to modulate antibody properties affecting drug development, such as thermodynamic stability, solubility or viscosity ("sequence optimization").

The amino acid modification includes, for example, deletions from and/or insertions into and/or substitutions of residues in the amino acid sequence of the binding molecule, preferably an antibody or an antigen-binding fragment. Any combination of deletions, insertions, and substitutions may be introduced into a "parent" amino acid sequence to arrive at a final variant. The amino acid modification also includes changes in post-translational processes of the binding molecule, such as changes in the number or position of glycosylation sites. For example, depending on the length of the CDRs and FRs themselves, 1, 2, 3, 4, 5 or 6 amino acids may be inserted into or deleted from each CDR, and 1, 2, 3, 4, 5, 6, 7, 8, 9, 10, 11, 12, 13, 14, 15, 16, 17, 18, 19, 20 or 25 amino acids may be inserted into or deleted from each FR. Insertion variants of the binding molecules (particularly the antibody or the antibody fragment) of the present disclosure include fusion products of an antibody or an antibody fragment with an enzyme or another functional polypeptide (e.g., which increases the serum half-life of the binding molecule (e.g., an antibody or an antibody fragment)). Moreover, amino acid substitutions may be introduced into the CDR, VH or FR regions of the heavy and/or light chain. Conservative substitutions are preferred, for example, they may be made on the basis of similarity in the polarity, charge, solubility, hydrophobicity, hydrophilicity and/or amphipathy property of the residues involved.

"Conservative substitution" refers to a substitution to another amino acid residue having similar properties to the original amino acid residue. For example, lysine, arginine and histidine have similar properties in that they have basic side chains, and aspartic acid and glutamic acid have similar properties in that they have acidic side chains. In addition, glycine, asparagine, glutamine, serine, threonine, tyrosine, cysteine and tryptophan have similar properties in that they have uncharged polar side chains, and alanine, valine, leucine, threonine, isoleucine, proline, phenylalanine and methionine have similar properties in that they have nonpolar side chains. In addition, tyrosine, phenylalanine, tryptophan and histidine have similar properties in that they have aromatic side chains. Thus, it will be apparent to those skilled in the art that even when an amino acid residue in a group exhibiting similar properties as described above is substituted, it will not exhibit a particular change in properties.

In addition to the CDRs and FRs, modifications may also be introduced into the Fc portion of the binding molecules (preferably an antibody or an antigen-binding fragment thereof). Such modifications may be used for modulating the functional properties of the antibody, for example, interaction with complement proteins (such as C1q and/or Fc receptors) on other immune cells, or for modulating serum half-life or antibody-dependent cell-mediated cytotoxicity (ADCC). Mutations that alter effector function may be introduced into the Fc domain by conventional methods known in the art. Exemplary modifications include: Asn297 → Ala297 and Asn297 → Gln297, or Lys322 → Ala322, and optionally Leu234 → Ala234 and Leu235 → Ala234 leading to IgG1 glycosylation, which have been reported to reduce or eliminate antibody-derived cell-mediated cytotoxicity (ADCC) and/or complement-dependent cytotoxicity (CDC). Examples of ways to prolong serum half-life include the addition of peptide or protein domains that bind to other proteins in the human body, such as serum albumin, immunoglobulin Fc region or neonatal Fc receptor (FcRn). Other conceivable ways to prolong serum half-life include: extension of the amino-terminus with polypeptide chains of varying lengths (e.g., XTEN technology or PASylation^{®}), conjugation to non-protein polymers including, but not limited to: various polyols, such as polyethylene glycol (PEGylation), polypropylene glycol, polyalkylene oxide, or a copolymer of polyethylene glycol with polypropylene glycol; or carbohydrates, such as hydroxyethyl starch (e.g., HESylation^{®}) or polysialic acid (e.g., PolyXen^{®}). Furthermore, as is known in the art, amino acid substitutions can be made at various positions in the binding molecules to facilitate the addition of the polymers.

The term "binding to FXI/FXIa" herein refers to the ability to interact with FXI/FXIa or an epitope thereof, wherein the FXI/FXIa or the epitope thereof may be derived from human.

The term "antigen-binding site" herein refers to a continuous or discontinuous three-dimensional spatial site on an antigen that is recognized by the antibody or the antigen-binding fragment of the present disclosure.

The term "epitope" refers to a site on an antigen to which an immunoglobulin or an antibody binds. An epitopes can be formed from contiguous amino acids, or non-contiguous amino acids that are brought into spatial proximity by tertiary folding of the protein. An epitope formed from contiguous amino acids are generally retained after exposure to a denaturing solvent, while an epitope formed by tertiary folding are generally lost after a denaturing solvent treatment. An epitope generally exists in a unique spatial conformation, and comprises, for example, at least 3-15 amino acids. Methods for determining an epitope are well known in the art and include an immunoblotting assay, an immunoprecipitation assay, and the like. Methods for determining the spatial conformation of an epitope include techniques in the art and techniques described herein, such as X-ray crystallography and two-dimensional nuclear magnetic resonance.

The term "specific binding" or "selective binding" refers to binding of an antibody to an epitope on a predetermined antigen. Generally, an antibody binds to a predetermined antigen or epitope thereof with an equilibrium dissociation constant (K_{D}) of about less than 10⁻⁷ M or even less and with an affinity that is at least twice as high as its affinity for binding to a non-specific antigen other than the predetermined antigen or the epitope thereof (or non-specific antigens other than closely related antigens, e.g., BSA, etc.), when determined by surface plasmon resonance (SPR) techniques in an instrument using recombinant human FXI/FXIa or an epitope thereof as the analyte and an antibody as the ligand.

"Affinity" refers to the overall strength of a non-covalent interaction between a single binding site of a molecule (e.g., an antibody) and its binding partner (e.g., an antigen). Unless otherwise indicated, as used herein, "binding affinity" refers to an internal binding affinity that reflects a 1:1 interaction between members of a binding pair (e.g., an antibody and an antigen). The affinity of a molecule X for its ligand Y can be generally expressed by the equilibrium dissociation constant (KD). Affinity can be determined by conventional methods known in the art, including those described herein. The term "kassoc" or "ka" refers to the association rate of a particular antibody-antigen interaction, while the term "kdis" or "kd" as used herein is intended to refer to the dissociation rate of a particular antibody-antigen interaction. As used herein, the term "KD" refers to the equilibrium dissociation constant, which is obtained from the ratio of kd to ka (i.e., kd/ka) and expressed as molar concentration (M). The KD values of antibodies can be determined by methods known in the art, for example, methods for determining antibody KD include measuring surface plasmon resonance using a biosensing system, such as a system, or measuring affinity in solution by solution equilibrium titration (SET) assay. The term "cross-reactivity" refers to the ability of the antibody (or the fragment thereof) of the present disclosure to bind to FXI/FXIa from different species. For example, the antibody of the present disclosure that binds to human FXI/FXIa may also bind to FXI/FXIa from another species. Cross-reactivity is determined by detecting specific reactivity with purified antigen in binding assays (e.g., SPR or ELISA) or binding or functional interactions with cells physiologically expressing FXI/FXIa. Methods for determining cross-reactivity include standard binding assays as described herein, for example, surface plasmon resonance analysis or flow cytometry.

The terms "inhibition" and "blocking" are used interchangeably and encompass partial and complete inhibition/blocking. Inhibition or blocking of FXI/FXIa preferably reduces or alters the normal level or type of activity that occurs when FXI/FXIa binding occurs without inhibition or blocking. Inhibition and blocking are also intended to include any measurable reduction in binding affinity for FXI/FXIa in contact with an anti-FXI/FXIa antibody as compared to FXI/FXIa not in contact with an anti-FXI/FXIa antibody.

The term "inhibition of growth" (e.g., involving cells) is intended to include any measurable reduction in cell growth.

Methods for producing and purifying antibodies and antigen-binding fragments are well known in the prior art and can be found in, for example, "Antibodies: A Laboratory Manual", Cold Spring Harbor Press (chapters 5-8 and 15). For example, mice can be immunized with human FXI/FXIA or a fragment thereof, and the resulting antibodies can be renatured and purified, and amino acid sequencing can be performed by conventional methods. Likewise, antigen-binding fragments can be prepared by conventional methods. The antibody or the antigen-binding fragment described herein is genetically engineered to contain one or more additional human FRs in the non-human-derived CDRs. Human FR germline sequences can be obtained from ImMunoGeneTics (IMGT) or from the Immunoglobulin Journal, 2001ISBN012441351. The engineered antibody or the antigen-binding fragment of the present disclosure can be prepared and purified by conventional methods. For example, cDNA sequences encoding the heavy and light chains can be cloned and recombined into an expression vector. The recombinant expression vector can stably transfect CHO cells. Mammalian expression systems may result in glycosylation of antibodies, particularly at the highly conserved N-terminus of the Fc region. Stable clones are obtained by expression of antibodies specifically binding to the human-derived antigen. Positive clones are expanded in a serum-free medium of a bioreactor to produce antibodies. The culture medium with the secreted antibody can be purified and collected by conventional techniques. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

The antibody disclosed herein refers to a monoclonal antibody (mAb), i.e., an antibody derived from a single clonal cell strain, which is not limited to eukaryotic, prokaryotic, or phage clonal cell strains. The monoclonal antibody or the antigen-binding fragment can be obtained by, for example, hybridoma technology, recombinant technology, phage display technology, synthetic technology (e.g., CDR-grafting), or other technologies known in the art.

Antibodies can be competitively screened for binding to the same epitope using conventional techniques known to those skilled in the art. For example, competition and cross-competition studies can be performed to obtain antibodies that compete or cross-compete with one another for binding to an antigen. A high-throughput method for obtaining antibodies that bind to the same epitope based on their cross-competition is described in International Patent Publication No. WO03/48731. Therefore, an antibody and an antigen-binding fragment thereof that competes with the antibody molecule of the present disclosure for binding to the same epitope on FXI/FXIa can be obtained by conventional techniques known to those skilled in the art.

"Giving", "administering" and "treating", when applied to animals, humans, experimental subjects, cells, tissues, organs or biological fluid, refer to contact of an exogenous drug, a therapeutic agent, a diagnostic agent or a composition with the animals, humans, subjects, cells, tissues, organs or biological fluid. "Giving", "administering" and "treating" can refer to, for example, therapeutic, pharmacokinetic, diagnostic, research, and experimental methods. The treatment of the cells comprises making the reagent in contact with the cells and making the reagent in contact with fluid, where the fluid is in contact with the cells. "Giving", "administering" and "treating" also refer to treating, e.g., a cell, by a reagent, diagnosis, a binding composition, or by another cell *in vitro* and *ex vivo.* "Treating", when applied to humans, veterinary or research subjects, refers to therapeutic treatment, preventive or prophylactic measures, and research and diagnostic applications.

"Treatment" refers to administering a therapeutic agent, such as a composition comprising any of the antibodies or the antigen-binding fragments thereof of the present disclosure, either internally or externally to a subject who has had, is suspected of having, or is predisposed to having one or more diseases or symptoms thereof on which the therapeutic agent is known to have a therapeutic effect. Generally, the therapeutic agent is administered in an amount effective to alleviate one or more symptoms of the disease in the subject or population being treated, whether by inducing regression of such symptoms or inhibiting the development of such symptoms into any clinically measurable degree. The amount of therapeutic agent effective to alleviate any particular symptom of the disease (also referred to as the "therapeutically effective amount") may vary depending on factors such as the disease state, age and weight of the subject, and the ability of the drug to produce a desired therapeutic effect in the subject. Whether a symptom of a disease has been alleviated can be evaluated by any clinical testing methods commonly used by doctors or other health care professionals to evaluate the severity or progression of the symptom. Although embodiments of the present disclosure (e.g., treatment methods or articles) may be ineffective in alleviating symptoms of a disease of interest in a certain subject, they shall alleviate the symptoms of the disease of interest in a statistically significant number of subjects as determined by any statistical test method known in the art, such as the Student's t-test, Chi-square test, U-test by Mann and Whitney, Kruskal-Wallis test (H-test), Jonckheere-Terpstra test and Wilcoxon test.

"Effective amount" comprises an amount sufficient to ameliorate or prevent a symptom of a medical disorder. An effective amount also refers to an amount sufficient to allow or facilitate diagnosis. The effective amount for a particular subject or veterinary subject may vary depending on the factors such as the disorder to be treated, the general health of the subject, the method and route and dosage of administration, and the severity of side effects. An effective amount can be the maximum dose or administration regimen to avoid significant side effects or toxic effects.

"Homology" or "identity" refers to sequence similarity between two polynucleotide sequences or between two polypeptides. When positions in two compared sequences are occupied by identical bases or amino acid monomer subunits, e.g., if the position of each of two DNA molecules is occupied by adenine, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. Generally, when two sequences are aligned, comparison is performed to obtain the maximum homology percentage.

"Cell", "cell line" and "cell culture" are used interchangeably, and all such designations include their progenies. It should also be understood that all progenies may not be precisely identical in DNA content due to deliberate or unintentional mutations. Mutant progeny with the same function or biological activity as screened in the original transformed cells is included.

The term "optional" or "optionally" means that the event or circumstance subsequently described may, but not necessarily, occur, and that the description includes instances where the event or circumstance occurs or does not occur. For example, "optionally comprising 1-3 antibody heavy chain variable regions" means that the antibody heavy chain variable region of a particular sequence may, but not necessarily, be present.

"Pharmaceutical composition" refers to a mixture containing one or more of the antibodies and the antigen-binding fragments described herein or a physiologically/pharmaceutically acceptable salt or pro-drug thereof, and other chemical components such as physiologically/pharmaceutically acceptable carriers and excipients. The pharmaceutical composition is intended to promote the administration to an organism to facilitate the absorption of the active ingredient, thereby exerting biological activities.

### Examples

The present disclosure is further described below with reference to examples, which, however, are not intended to limit the scope of the present disclosure.

Experimental procedures without specific conditions indicated in the examples or test examples are generally conducted according to conventional conditions, or according to conditions recommended by the manufacturer of the starting materials or commercial products, see Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents without specific origins indicated are commercially available conventional reagents.

### Example 1. Preparation of Human FXI/FXIa Antigen and Detection Protein

### 1. Human FXI/FXIa protein

Human FXI/FXIa protein (Uniprot Acc No. P03951) was purchased from Enzyme research laboratories (FXI: Cat. HFXI 1111; FXIa: HFXIa 1111a) as an antigen and a protein for detection involved in the present disclosure. Unless otherwise specified, the following FXI/FXIa antigen was a human FXI/FXIa antigen.

### > Amino acid sequence of human FXI/FXIa:

ECVTQLLKDTCFEGGDITTVFTPSAKYCQVVCTYHPRCLLFTFTAESPSEDPTRW
FTCVLKDSVTETLPRVNRTAAISGYSFKQCSHQISACNKDIYVDLDMKGINYNSS
VAKSAQECQERCTDDVHCHFFTYATRQFPSLEHRNICLLKHTQTGTPTRITKLDK
VVSGFSLKSCALSNLACIRDIFPNTVFADSNIDSVMAPDAFVCGRICTHHPGCLFF
TFFSQEWPKESQRNLCLLKTSESGLPSTRIKKSKALSGFSLQSCRHSIPVFCHSSF
YHDTDFLGEELDIVAAKSHEACQKLCTNAVRCQFFTYTPAQASCNEGKGKCYL
KLSSNGSPTKILHGRGGISGYTLRLCKMDNECTTKIKPRIVGGTASVRGEWPWQ

VTLHTTSPTQRHLCGGSIIGNQWILTAAHCFYGVESPKILRVYSGILNQSEIKEDT
SFFGVQEIIIHDQYKMAESGYDIALLKLETTVNYTDSQRPICLPSKGDRNVIYTD
CWVTGWGYRKLRDKIQNTLQKAKIPLVTNEECQKRYRGHKITHKMICAGYRE
GGKDACKGDSGGPLSCKHNEVWHLVGITSWGEGCAQRERPGVYTNVVEYVD
WILEKTQAV (SEQ ID NO: 1).

The following KLH-conjugated FXIa-specific polypeptides were synthesized for immunization of mice.
KLH-CFYGVESPKILRVYSGIL (SEQ ID NO: 2)
KLH-CGYRKLRDKIQNTLQKAKIPL (SEQ ID NO: 3)
KLH-CGVQEIIIHDQYKMAESGYDI (SEQ ID NO: 4)

### 2. Purification of FXI/FXIa-related recombinant protein and purification of hybridoma antibody and recombinant antibody

(1) Separation and purification/ProteinG affinity chromatography of mouse hybridoma supernatant:
   For the purification of mouse hybridoma supernatant, ProteinG packing material (KANEKA: Cat. KanCapTMG) was preferred for affinity chromatography. The cultured hybridoma was centrifuged to obtain the supernatant, which was then purified by a gravity column packed with ProteinG packing material. Firstly, the column was regenerated with 3-5 column volumes of 6 M guanidine hydrochloride (Sigma: Cat. G3272-1KG), and washed with 3-5 column volumes of pure water; the column was equilibrated with 3-5 column volumes of 1× PBS (pH 7.4) (Sangon Biotech (Shanghai) Co., Ltd.: Cat. E607016-0500) buffer system as an equilibration buffer; the supernatant was loaded on the column at a low flow rate for binding, with the flow rate controlled to allow a retention time of about 1 min or longer; the column was washed with 3-5 column volumes of 1× PBS (pH 7.4) until the UV absorbance fell back to baseline; the sample was eluted with a 0.1 M Glycine (pH 3.0) (Sigma: Cat. 410225-250G) buffer, the elution peaks were collected by UV detection, and the eluted product was rapidly adjusted to pH 7-8 with 1 M Tris-HCl (pH 8.0) (Vetec, Cat. V900483) and temporarily stored. For the eluted product, solution displacement may be performed by methods well known to those skilled in the art, such as ultrafiltration concentration using an ultrafiltration tube to replace the solution to a desired buffer system, or replacement with a desired buffer system by size exclusion (e.g., G-25 desalination).
(2) Extraction of the human Fc-tagged fusion protein or antibody by Protein A affinity chromatography:
   Firstly, the cell culture expressing the Fc fusion protein or antibody centrifuged at a high speed to obtain the supernatant. A Protein A (GE, Cat: 17-5474-99) affinity column was regenerated with 5 column volumes of 0.1 M NaOH (Sigma: Cat: 71687-500g), and washed and equilibrated with 5 column volumes of 1× PBS (pH 7.4) (Sangon Biotech (Shanghai) Co., Ltd.: Cat. E607016-0500). The supernatant was loaded at a low flow rate for binding, with the flow rate controlled to allow a retention time of about 1 min or longer. After the binding was completed, the column was washed with 5 column volumes of 1× PBS (pH 7.4) until the UV absorbance fell back to baseline. The sample was eluted with a 0.1 M Glycine (pH 3.0) (Sigma: Cat. 410225-250G) buffer, the elution peaks were collected by UV detection, and the eluted product was rapidly adjusted to pH 7-8 with 1 M Tris-HCl (pH 8.0) (Vetec, Cat. V900483) and temporarily stored. For the eluted product, solution displacement may be performed by methods well known to those skilled in the art, such as ultrafiltration concentration using an ultrafiltration tube to replace the solution to a desired buffer system, or replacement with a desired buffer system by size exclusion (e.g., G-25 desalination).
(3) Polish purification by anion chromatography:
   Firstly, the sample was diluted with an equilibration buffer (20 mM Tris pH 8.0 (Vetec, Cat. V900483)) to allow the conductivity to be less than 3 ms/cm, and the Q HP (GE, Cat: 17-1014-01) anion chromatography column was regenerated with 5 column volumes of 0.5 M NaOH (Sigma Cat: 71687-500g), and washed and equilibrated with 15 column volumes of 20 mM Tris pH 8.0 (Vetec, Cat. V900483). The supernatant was loaded at a low flow rate for binding, with the flow rate controlled to allow a retention time of about 2 min or longer. After the binding was completed, the column was washed with 10 column volumes of 20mM Tris pH 8.0 (Vetec, Cat. V900483) until the UV absorbance fell back to baseline. 0-100% gradient elution was performed with an elution buffer (20 mM Tris pH 8.0 (Vetec, Cat. V900483)) and 0.5 M NaCl (Vetec, Cat. V900058); elution peaks were detected and collected according to SEC-UPLC (Column: Waters ACQUITY UPLC^{®} Protein BEH SEC column 200Å, 1.7 µm, Cat. 186005225) purity. For the eluted product, solution displacement may be performed by methods well known to those skilled in the art, such as ultrafiltration concentration using an ultrafiltration tube to replace the solution to a desired buffer system, or replacement with a desired buffer system by size exclusion (e.g., G-25 desalination).

### Example 2. Screening for Antibodies that Specifically Bind to FXI/FXIa

The antibodies having high affinity for FXIa were obtained by screening a fully human single-chain antibody phage library. 100 µg of Dynabeads MyOne Streptavidin T1 was bound to 2 µg of randomly biotinylated FXIa protein at room temperature for 1 h. The system was washed with PBST (0.05% Tween-20) 3 times, and skim milk was dissolved in 1× PBS to make a final concentration of 2% and used as a blocking solution, which was then added to the system for blocking at room temperature for 1 h. A fully human single-chain antibody phage display library blocked with 2% milk at room temperature for 1 h was added and reacted at room temperature for 1 h. The system was washed with a PBST (0.05% Tween-20), pH 7.4 solution 11 times, with 20 up/down flips each time, to remove unbound phages. The remaining phages specifically binding to FXIa were eluted with 0.5 mL of 1 mg/mL trypsin, and separately used to infect *E. coli* TG1 in the logarithmic growth phase, and phages were generated and purified for the next round of screening. The same screening process was repeated for 2-3 rounds with the amount of FXIa gradually decreased to 0.5 µg (for the second round) and to 0.1 µg (for the third round). After the third round of screening, positive clones were enriched.

From the selected and enriched clones, monoclonal colonies were picked and packaged into single-chain antibody phages for phage ELISA testing. The ELISA plates were coated with 1 µg/mL FXIa protein, left to stand at 4 °C overnight, washed with PBST (0.05% Tween-20) 3 times, blocked with 2% skim milk for 1 h at room temperature, and washed with PBST (0.05% Tween-20) 3 times. After that, the phage supernatant diluted with a blocking solution was added and reacted at room temperature for 1 h, and the plate was washed with PBST (0.05% Tween-20) 6 times. The anti-M13 HRP (Sino Biological Inc., 11973-MM05T-H) was added and reacted at room temperature for 1 h, and the plate was washed with PBST (0.05% Tween-20) 3 times. 100 µL of TMB chromogenic substrate was added, and 100 µL of 1 M sulfuric acid was added to stop the reaction. The absorbance values at 450 nm were read with a SpectraMax M5 microplate reader for the test. Clones with OD₄₅₀ values greater than 3 times the background value in the ELISA binding test was sequenced to obtain the high-affinity single-chain antibodies.

### Example 3. Construction of Intact Anti-FXI/FXIa Monoclonal Antibodies

The high-affinity specific sequences obtained by screening in Example 2 were used for constructing intact recombinant antibodies. Then, an ELISA binding assay, a ForteBio protein interaction assay and an FXIa enzyme activity inhibition assay were performed to determine that two of the antibodies had strong binding ability and were able to effectively inhibit the FXIa enzyme activity. The intact variable region sequences are as follows:
> 0012-VH: 
QVQLQQSGAEVKKPGASVKVSCKVSGYTLTELSMHWVRQAPGKGLEWMGGF
DPEDGETIYAQKFQGRVTMTEDTSTDTAYMELSSLRSEDTAVYYCATDPHRTW
WRYFDWLYPRGMDVWGQGTTVTVSS
SEQ ID NO: 5
> 0012-VL:

EIVMTQSPGTLSLSPGERATLSCRASQTVGKNYLAWYQQKPGQAPRLLIYGASN
RATGIPDRFSGSGSGTDFTLTISRLEPEDFGTYYCQQFRSYPYTFGQGTKVEIK
SEQ ID NO: 6

(Note: the numbering scheme for the antibody was kabat).

In the sequences of the heavy chain and light chain variable regions of the antibody, the regions were arranged in the following order: FR1-CDR1-FR2-CDR2-FR3-CDR3-FR4, with the underlined portions being CDR1, CDR2 and CDR3 sequences, respectively. The CDR sequences of each of the heavy and light chains are summarized in Table 1.

**Table 1. CDR sequences of heavy and light chains**

| Antibody No. | Heavy chain | | Light chain | |
|---|---|---|---|---|
| 0012 | HCDR1 | ELSMH (SEQ ID NO: 7) | LCDR1 | RASQTVGKNYLA (SEQ ID NO: 10) |
| | HCDR2 | GFDPEDGETIYAQKFQG (SEQ ID NO: 8) | LCDR2 | GASNRAT (SEQ ID NO: 11) |
| | HCDR3 | DPHRTWWRYFDWLYPRGMDV (SEQ ID NO: 9) | LCDR3 | QQFRSYPYT (SEQ ID NO: 12) |

### Example 4. Affinity Maturation of Anti-FXI/FXIa Monoclonal Antibodies

Antibody molecule 0012 was subjected to three-dimensional structure modeling, which mimics the binding to a known antigenic structure (PDB ID: 6AOD, Chain: C). Part of amino acid residues were selected by referring to mutation hot spots of human germline genes, three-dimensional structure and binding simulation results to establish several random mutation phage libraries. Functional antibodies with improved affinity were selected by using a phage library display technology. The new amino acid residues obtained from different libraries were combined and verified to obtain functional antibodies with improved affinity and function. The sequences of the heavy chain and light chain variable regions of the obtained antibody molecules are as follows:
> F-VH: 
QVQLQQSGAEVKKPGASVKVSCKVSGYTLTSIFMHWVRQAPGKGLEWMGGF
DPEDGETRYAQKFQGRVTMTEDTSTDTAYMELSSLRSEDTAVYYCATDPHRTW
WRYFDWLYPRGMDVWGQGTTVTVSS
SEQ ID NO: 17
> H-VH: 
QVQLQQSGAEVKKPGASVKVSCKVSGYTLTGLLMHWVRQAPGKGLEWMGGF
DPQDGETVYAQKFQGRVTMTEDTSTDTAYMELSSLRSEDTAVYYCATDPHRTW
WRYFDWLYPRGMDVWGQGTTVTVSS
SEQ ID NO: 18
> J-VH: 
QVQLQQSGAEVKKPGASVKVSCKVSGYTLTGVLMHWVRQAPGKGLEWMGGF
DPEDGETVYAQKFQGRVTMTEDTSTDTAYMELSSLRSEDTAVYYCATDPHRTW
WRYFDWLYPRGMDVWGQGTTVTVSS
SEQ ID NO: 19
> K-VH: 
QVQLQQSGAEVKKPGASVKVSCKVSGYTLTSILMHWVRQAPGKGLEWMGGF
DPQDGETVYAQKFQGRVTMTEDTSTDTAYMELSSLRSEDTAVYYCATDPHRTW
WRYFDWLYPRGMDVWGQGTTVTVSS
SEQ ID NO: 20
> 3-VL:

EIVMTQSPGTLSLSPGERATLSCRASQTVGKNYLAWYQQKPGQAPRLLIYEASV
RALGIPDRFSGSGSGTDFTLTISRLEPEDFGTYYCQQFRSYPYTFGQGTKVEIK
SEQ ID NO: 21

The numbering scheme for the above sequences was Kabat, the regions were arranged in the following order: FR1-CDR1-FR2-CDR2-FR3-CDR3-FR4, with the underlined portions in the sequence being CDR1, CDR2 and CDR3, respectively.

The heavy chain and light chain variable regions of 0012-F3 were F-VH and 3-VL, respectively; the heavy chain and light chain variable regions of 0012-H3 were H-VH and 3-VL, respectively; the heavy chain and light chain variable regions of 0012-J3 were J-VH and 3-VL, respectively; and the heavy chain and light chain variable regions of 0012-K3 (i.e., 1209) were K-VH and 3-VL, respectively.

**Table 2. CDR sequences of the heavy and light chains of antibody 0012 after affinity maturation**

| No. | Sequences of heavy chain variable region (VH) and light chain variable region (VL) | |
|---|---|---|
| F-VH | HCDR1 | SIFMH (SEQ ID NO: 22) |
| | HCDR2 | GFDPEDGETRYAQKFQG (SEQ ID NO: 23) |
| | HCDR3 | DPHRTWWRYFDWLYPRGMDV (SEQ ID NO: 9) |
| H-VH | HCDR1 | GLLMH (SEQ ID NO: 24) |
| | HCDR2 | GFDPODGETVYAOKFOG (SEQ ID NO: 25) |
| | HCDR3 | DPHRTWWRYFDWLYPRGMDV (SEQ ID NO: 9) |
| J-VH | HCDR1 | GVLMH (SEQ ID NO: 26) |
| | HCDR2 | GFDPEDGETVYAQKFQG (SEQ ID NO: 27) |
| | HCDR3 | DPHRTWWRYFDWLYPRGMDV (SEQ ID NO: 9) |
| K-VH | HCDR1 | SILMH (SEQ ID NO: 28) |
| | HCDR2 | GFDPODGETVYAOKFOG (SEQ ID NO: 25) |
| | HCDR3 | DPHRTWWRYFDWLYPRGMDV (SEQ ID NO: 9) |
| 3-VL | LCDR1 | RASQTVGKNYLA (SEQ ID NO: 10) |
| | LCDR2 | EASVRAL (SEQ ID NO: 29) |
| | LCDR3 | QQFRSYPYT (SEQ ID NO: 12) |

### Example 5. Engineering to Reduce Immunogenicity of Anti-FXI/FXIa Fully Human Monoclonal Antibodies

By three-dimensional structure homologous modeling of selected fully human specific antibody molecules, in combination with the results of comparison with a V-base human germline sequence database and an IMGT human antibody heavy chain variable region germline gene database, heavy chain and light chain variable region germline genes with high homology with the screened antibodies were selected as templates, and the FR region and the CDR region of the original monoclonal antibody was engineered to make the sequences closer the human germline genes while retaining their functions. Three-dimensional structure modeling and analysis were performed on the grafted antibodies, and back mutations were performed on the specific sites in the FR regions that affected the structure and morphology of the CDR regions. The amino acid residues were identified using the Kabat numbering scheme and annotated. The engineered antibodies have higher stability and lower immunogenicity.

Selection of architectures of affinity-matured antibody germline genes

After analysis, for antibody 0012, the human germline gene VH1-24 in the Vbase database was used as the heavy chain template and the human germline gene VKIIIA27 in the Vbase database was used as the light chain template.

After genetic engineering, antibodies Fg3g (i.e., 1268), Hg3g, Jg3g and Kg3g (i.e., 1267) were obtained. The sequences of the heavy chain and light chain variable regions of these antibodies are as follows:
> VH of Fg3g: 
QVQLVQSGAEVKKPGASVKVSCKVSGYTLTSIFMHWVRQAPGKGLEWMGGFD
PEDGETRYAQKFQGRVTMTEDTSTDTAYMELSSLRSEDTAVYYCATDPHRTWW
RYFDWLYPRGMDVWGQGTTVTVSS
SEQ ID NO: 30
> VH of Hg3g: 
QVQLVQSGAEVKKPGASVKVSCKVSGYTLTGLLMHWVRQAPGKGLEWMGGF
DPQDGETVYAQKFQGRVTMTEDTSTDTAYMELSSLRSEDTAVYYCATDPHRTW
WRYFDWLYPRGMDVWGQGTTVTVSS
SEQ ID NO: 31
> VH of Jg3g: 
QVQLVQSGAEVKKPGASVKVSCKVSGYTLTGVLMHWVRQAPGKGLEWMGGF
DPEDGETVYAQKFQGRVTMTEDTSTDTAYMELSSLRSEDTAVYYCATDPHRTW
WRYFDWLYPRGMDVWGQGTTVTVSS
SEQ ID NO: 32
>VH of Kg3g: 
QVQLVQSGAEVKKPGASVKVSCKVSGYTLTSILMHWVRQAPGKGLEWMGGFD
PQDGETVYAQKFQGRVTMTEDTSTDTAYMELSSLRSEDTAVYYCATDPHRTWW
RYFDWLYPRGMDVWGQGTTVTVSS
SEQ ID NO: 33
> VL of Fg3g or Hg3g or Jg3g or Kg3g:

EIVLTQSPGTLSLSPGERATLSCRASQTVGKNYLAWYQQKPGQAPRLLIYEASVR
ALGIPDRFSGSGSGTDFTLTISRLEPEDFAVYYCQQFRSYPYTFGQGTKVEIK
SEQ ID NO: 34

The CDR numbering scheme for the above antibodies was Kabat. The regions were arranged in the following order: FR1-CDR1-FR2-CDR2-FR3-CDR3-FR4, with the underlined portions in the sequence being CDR1, CDR2 and CDR3, respectively.

Each of the heavy chain variable regions was fused with CH1 (SEQ ID NO: 13) of the corresponding human antibody, then fused with IgG1 Fc (SEQ ID NO: 67), and the light chain variable region was fused with human κ (SEQ ID NO: 14) to form a recombinant antibody for subsequent assay.
> CH1 of human antibody: 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKRV
SEQ ID NO: 13
> IgG1Fc of human antibody: 
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMH
EALHNHYTQKSLSLSPGK
SEQ ID NO: 67
> light chain Cκ of human antibody:



SEQ ID NO: 14

Sequences of full length heavy and light chains of the antibody exemplified in 1209:
> 1209-HC: 
QVQLQQSGAEVKKPGASVKVSCKVSGYTLTSILMHWVRQAPGKGLEWMGGF
DPQDGETVYAQKFQGRVTMTEDTSTDTAYMELSSLRSEDTAVYYCATDPHRTW
WRYFDWLYPRGMDVWGQGTTVTVSSASTKGPSVFPLAPSSKSTSGGTAALGCL
VKDYFPEPVTVSWNSGALTSGVHTFPAVLQSSGLYSLSSVVTVPSSSLGTQTYIC
NVNHKPSNTKVDKRVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSREEMTK
NQVSLTCLVKGFYPSDIAVEWESNGQPENNYKTTPPVLDSDGSFFLYSKLTVDKS
RWQQGNVFSCSVMHEALHNHYTQKSLSLSPGK
SEQ ID NO: 15
> 1209-LC:

EIVMTQSPGTLSLSPGERATLSCRASQTVGKNYLAWYQQKPGQAPRLLIYEASV
RALGIPDRFSGSGSGTDFTLTISRLEPEDFGTYYCQQFRSYPYTFGQGTKVEIKRT
VAAPSVFIFPPSDEQLKSGTASVVCLLNNFYPREAKVQWKVDNALQSGNSQESV

TEQDSKDSTYSLSSTLTLSKADYEKHKVYACEVTHQGLSSPVTKSFNRGEC
SEQ ID NO: 16

### Example 6. Screening and Preparation of Anti-FXI/FXIa Hybridoma Monoclonal Antibodies

### 1. Immunization of mice

Shanghai ChemPartner was entrusted to perform the immunization and splenocyte fusion experiments. 5 SJL white mice and 5 Balb/c white mice were each immunized with a mixture of 25 µg of FXIa antigen and 3 KLH-conjugated polypeptides, and an adjuvant. The immunization was performed on day 0, day 14 and day 35. On day 0, 25 µg of emulsified antigen was injected intraperitoneally (IP) in each mouse. On day 14 and day 35, 12.5 µg of emulsified antigen was injected. Blood was collected on day 21 and day 42, and the antibody titer in mouse serum was determined by ELISA. After 4-5 immunizations, mice in which the antibody titer in serum was high and was reaching a plateau were selected for splenocyte fusion. The booster immunization was performed 3 days before the splenocyte fusion, and 50 µg of antigen solution in normal saline was intraperitoneally (IP) injected.

### 2. Splenocyte fusion

Spleen lymphocytes and myeloma cells Sp2/0-Ag14 were fused by following an optimized PEG-mediated fusion procedure to obtain hybridoma cells. The fused hybridoma cells were seeded in a 96-well plate at 1×10⁴ to 1×10⁵ cells per well, and the plate was incubated at 37 °C with 5% CO₂, supplemented with an HAT complete medium at 100 µL/well, and assayed by ELISA 10-14 days later.

### 3. Screening of hybridoma cells

Hybridoma culture supernatants were assayed by a binding ELISA method according to the growth density of hybridoma cells. The supernatant of positive well cells assayed by binding ELISA were subjected to purification, a cell binding experiment and a cell blocking experiment. Well cells with positive results for both binding and blocking experiments were promptly expanded and frozen and sequenced.

### 4. Further screening of hybridoma cells

Hybridoma culture supernatants were assayed by a binding ELISA method according to the growth density of hybridoma cells. An enzyme activity inhibition experiment was performed on the supernatant of positive well cells assayed by the binding ELISA. Well cells with positive results for both binding and function experiments were subjected to one to two subcloning dilutions until single cell clones were obtained.

FXI/FXIa binding ELISA and FXIa enzyme activity inhibition assays were also performed on cells in each subcloning. Hybridoma clones were obtained by the above screening process and further expanded to prepare antibodies, which were purified according to the purification example for use in the assay example.

### 5. Sequencing of hybridoma positive clones

Hybridoma cells in the logarithmic growth phase were collected, RNA was extracted using an RNeasy Micro kit (Qiagen, Cat No. 74004) following the procedures in the kit instructions, and reverse transcription was performed using a PrimeScript^{™} Reverse Transcriptase kit (Takara, Cat No. 2680A). The cDNA obtained by the reverse transcription was amplified by PCR using mouse Ig-Primer Set (Novagen, TB326 Rev.B 0503) and then sent for sequencing. A positive clone 3807 was obtained. The corresponding amino acid sequences of variable regions of the antibody are as follows:
> 3807-VH: 
EVQLQQSGAELVRPGTSVKLSCIASGFNIKDDYMHWVKQRPEQGLEWIGWIDP
ENGDTEYASKFQGKATITADTSANTAYLQFSSLTSEDTAVYYCLYGNFYYFDYW
GQGTTLTVSS
SEQ ID NO: 35
> 3807-VL:

QIVLTQSPAIMSASPGEKVTMTCSASSSINYMHWYQQKPGTSPKRWIYDTSKLA
SGVPARFSGSGSGTSYSLTISSMEAEDAATYYCHQRSFSPLTFGAGTKLELK
SEQ ID NO: 36

**Table 3. CDR sequences of heavy and light chains of antibody 3807**

| Heavy chain | | Light chain | |
|---|---|---|---|
| HCDR1 | DDYMH (SEQ ID NO: 37) | LCDR1 | SASSSINYMH (SEQ ID NO: 40) |
| HCDR2 | WIDPENGDTEYASKFQG (SEQ ID NO: 38) | LCDR2 | DTSKLAS (SEQ ID NO: 41) |
| HCDR3 | GNFYYFDY (SEQ ID NO: 39) | LCDR3 | HQRSFSPLT (SEQ ID NO: 42) |

### Example 7. Humanization of Anti-FXI/FXIa Hybridoma Antibodies

By three-dimensional structure homologous modeling of the antibody molecule 3807, in combination with the results of comparison with a V-base human germline sequence database and an IMGT human antibody heavy chain variable region germline gene database, heavy chain variable region germline genes with high homology with the screened antibodies were selected as templates, and CDRs of the mouse-derived monoclonal antibodies were grafted into corresponding human-derived modules. Three-dimensional structure modeling and analysis were performed on the grafted antibodies, and back mutations were performed on the specific sites in the FR regions that affected the structure and morphology of the CDR regions. The amino acid residues were identified using the Kabat numbering scheme and annotated.

### 1. Selection of humanized architectures of hybridoma clone 3807

For the murine antibody 3807, the humanized light chain template was IGKV3-11*01, and the heavy chain template was IGHV1-69-2*01. The humanized variable region sequences are as follows (with the underlined portions being CDRs):
> 3807 VH-CDR grafting 
QVQLVQSGAEVKKPGSSVKVSCKASGYTFTDDYMHWVRQAPGQGLEWMGWI
DPENGDTEYASKFQGRVTITADESTSTAYMELSSLRSEDTAVYYCARGNFYYFD
YWGQGTTVTVSS
SEQ ID NO: 43
> 3807 VL-CDR grafting

EIVLTQSPATLSLSPGERATLSCSASSSINYMHWYQQKPGQAPRLLIYDTSKLASG
IPARFSGSGSGTDFTLTISSLEPEDFAVYYCHQRSFSPLTFGQGTKLEIK

SEQ ID NO: 44

### 2. Back-mutation of hybridoma clone 3807

The sites and mutation patterns were designed as shown in Table 4.

**Table 4. Sequences of each of the heavy chain and light chain variable regions**

| 3807-VH | |
|---|---|
| 3807-VH1 | Y27F, T28N, F29I, T30K, A93L, R94Y |
| 3807-VH2 | Y27F, T28N, F29I, T30K, A93L, R94Y, E73T |
| 3807-VH3 | Y27F, T28N, F29I, T30K, A93L, R94Y, E73T, V67A |
| 3807-VH4 | Y27F, T28N, F29I, T30K, A93L, R94Y, E73T, R66K, V67A |
| 3807-VH5 | Y27F, T28N, F29I, T30K, A93L, R94Y, E73T, T75A, T76N |

| 3807-VL | |
|---|---|
| 3807-VL1 | No back mutation |
| 3807-VL2 | L47W, I58V |
| 3807-VL3 | R45K, L46R, L47W, I58V |
| 3807-VL4 | L47W, I58V, F71Y |

| | |
|---|---|
| The specific sequences are as follows. | |

> 3807-VH1: 
QVQLVQSGAEVKKPGSSVKVSCKASGFNIKDDYMHWVRQAPGQGLEWMGWI
DPENGDTEYASKFQGRVTITADESTSTAYMELSSLRSEDTAVYYCLYGNFYYFDY
WGQGTTVTVSS
SEQ ID NO: 45
> 3807-VH2: 
QVQLVQSGAEVKKPGSSVKVSCKASGFNIKDDYMHWVRQAPGQGLEWMGWI
DPENGDTEYASKFQGRVTITADTSTSTAYMELSSLRSEDTAVYYCLYGNFYYFDY
WGQGTTVTVSS
SEQ ID NO: 46
> 3807-VH3: 
QVQLVQSGAEVKKPGSSVKVSCKASGFNIKDDYMHWVRQAPGQGLEWIGWID
PENGDTEYASKFQGRATITADTSTSTAYMELSSLRSEDTAVYYCLYGNFYYFDY
WGQGTTVTVSS
SEQ ID NO: 47
> 3807-VH4: 
QVQLVQSGAEVKKPGSSVKVSCKASGFNIKDDYMHWVRQAPGQGLEWIGWID
PENGDTEYASKFQGKATITADTSTSTAYMELSSLRSEDTAVYYCLYGNFYYFDY
WGQGTTVTVSS
SEQ ID NO: 48
> 3807-VH5: 
QVQLVQSGAEVKKPGSSVKVSCKASGFNIKDDYMHWVRQAPGQGLEWMGWI
DPENGDTEYASKFQGRVTITADTSANTAYMELSSLRSEDTAVYYCLYGNFYYFD
YWGQGTTVTVSS
SEQ ID NO: 49
> 3807-VL1: EIVLTQSPATLSLSPGERATLSCSASSSINYMHWYQQKPGQAPRLLIYDTSKLASG 
IPARFSGSGSGTDFTLTISSLEPEDFAVYYCHQRSFSPLTFGQGTKLEIK
SEQ ID NO: 44
> 3807-VL2: 
EIVLTQSPATLSLSPGERATLSCSASSSINYMHWYQQKPGQAPRLWIYDTSKLAS
GVPARFSGSGSGTDFTLTISSLEPEDFAVYYCHQRSFSPLTFGQGTKLEIK
SEQ ID NO: 50
> 3807-VL3: 
EIVLTQSPATLSLSPGERATLSCSASSSINYMHWYQQKPGQAPKRWIYDTSKLAS
GVPARFSGSGSGTDFTLTISSLEPEDFAVYYCHQRSFSPLTFGQGTKLEIK
SEQ ID NO: 51
> 3807-VL4: 
EIVLTQSPATLSLSPGERATLSCSASSSINYMHWYQQKPGQAPRLWIYDTSKLAS
GVPARFSGSGSGTDYTLTISSLEPEDFAVYYCHQRSFSPLTFGQGTKLEIK
SEQ ID NO: 52
For antibody 3871, the VH was 3807-VH1 and the VL was 3807-VL3; and for antibody 3875, the VH was 3807-VH5 and the VL was 3807-VL3.

### Example 8. Engineering to Reduce Immunogenicity of Anti-FXI/FXIa Humanized Monoclonal Antibodies

3871 and 3875 were genetically engineered according to the method in Example 5 to provide antibodies with higher stability and lower immunogenicity. Only the heavy chain FR regions were engineered, the light chain variable region was unchanged.
1. The sequences of the genetically engineered heavy chain variable regions of the humanized antibody 3875 are as follows (light chain unchanged):
   > 3807-VH5 (ESAN) 
QVQLVQSGAEVKKPGSSVKVSCKASGFNIKDDYMHWVRQAPGQGLEWMGWI
DPENGDTEYASKFQGRVTITADESANTAYMELSSLRSEDTAVYYCLYGNFYYFD
YWGQGTTVTVSS
SEQ ID NO: 53
   > 3807-VH5 (TSTN) 
QVQLVQSGAEVKKPGSSVKVSCKASGFNIKDDYMHWVRQAPGQGLEWMGWI
DPENGDTEYASKFQGRVTITADTSTNTAYMELSSLRSEDTAVYYCLYGNFYYFD
YWGQGTTVTVSS
SEQ ID NO: 54
   > 3807-VH5 (ESTN) 
QVQLVQSGAEVKKPGSSVKVSCKASGFNIKDDYMHWVRQAPGQGLEWMGWI
DPENGDTEYASKFQGRVTITADESTNTAYMELSSLRSEDTAVYYCLYGNFYYFD
YWGQGTTVTVSS
SEQ ID NO: 55
2. The sequences of the genetically engineered heavy chain variable regions of the humanized antibody 3871 are as follows (light chain unchanged):
   > 3807-VH1 (AR) 
QVQLVQSGAEVKKPGSSVKVSCKASGFNIKDDYMHWVRQAPGQGLEWMGWI
DPENGDTEYASKFQGRVTITADESTSTAYMELSSLRSEDTAVYYCARGNFYYFD
YWGQGTTVTVSS
SEQ ID NO: 56
   > 3807-VH1 (YTFT) 
QVQLVQSGAEVKKPGSSVKVSCKASGYTFTDDYMHWVRQAPGQGLEWMGWI
DPENGDTEYASKFQGRVTITADESTSTAYMELSSLRSEDTAVYYCLYGNFYYFDY
WGQGTTVTVSS
SEQ ID NO: 57
   > 3807-VH1 (GTFS)

QVQLVQSGAEVKKPGSSVKVSCKASGGTFSDDYMHWVRQAPGQGLEWMGWI
DPENGDTEYASKFQGRVTITADESTSTAYMELSSLRSEDTAVYYCLYGNFYYFDY
WGQGTTVTVSS
SEQ ID NO: 58

For the antibody 3882, the VH was 3807-VH1 (GTFS) and the VL was 3807-VL3.

Each of the heavy chain variable regions was fused with the heavy chain CH1 (SEQ ID NO: 59) of the corresponding human antibody and the Fc (containing the S241P mutation) (SEQ ID NO: 60) of the human antibody IgG4, and the light chain variable region was fused with the constant region CL1 (SEQ ID NO: 14) of human κ to form a recombinant chimeric antibody for subsequent assay.
> Heavy chain CH1 of human antibody: 
ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRV
SEQ ID NO: 59
> IgG4P Fc (i.e., containing the S241P mutation) of human antibody:

ESKYGPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLGK
SEQ ID NO: 60

Full length sequences of the antibody exemplified in 3882:
> 3882-HC: 
QVQLVQSGAEVKKPGSSVKVSCKASGGTFSDDYMHWVRQAPGQGLEWMGWI
DPENGDTEYASKFQGRVTITADESTSTAYMELSSLRSEDTAVYYCLYGNFYYFDY
WGQGTTVTVSSASTKGPSVFPLAPCSRSTSESTAALGCLVKDYFPEPVTVSWNS
GALTSGVHTFPAVLQSSGLYSLSSVVTVPSSSLGTKTYTCNVDHKPSNTKVDKR
VESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQKSLSLSLGK
 SEQ ID NO: 61
> 3882-LC:

EIVLTQSPATLSLSPGERATLSCSASSSINYMHWYQQKPGQAPKRWIYDTSKLAS
GVPARFSGSGSGTDFTLTISSLEPEDFAVYYCHQRSFSPLTFGQGTKLEIKRTVAAP
SVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC
SEQ ID NO: 62

**Table 5. General formula of CDRs of the antibodies of the present disclosure**

| Heavy chain | | Light chain | |
|---|---|---|---|
| HCDR1 | X₁X₂X₃MH (SEQ ID NO: 63), wherein X₁ is selected from the group consisting of E, S, G and D, X₂ is selected from the group consisting of L, I, V and D, and X₃ is selected from the group consisting of S, F, L and Y | LCDR1 | RASQTVGKNYLA (SEQ ID NO: 10) or SASSSINYMH (SEQ ID NO: 40) |
| HCDR2 | X₄X₅DPX₆X₇GX₈TX₉YAX₁₀KFQG (SEQ ID NO: 64), wherein X₄ is selected from the group consisting of G and W, X₅ is selected from the group consisting of F and 1, X₆ is selected from the group consisting of E and Q, X₇ is selected from the group consisting of D and N, X₈ is selected from the group consisting of E and D, X₉ is selected from I, R, V and E, and X₁₀ is selected from the group consisting of Q and S | LCDR2 | X₁₁X₁₂SX₁₃X₁₄AX₁₅ (SEQ ID NO: 65), wherein X₁₁ is selected from the group consisting of G, E and D, X₁₂ is selected from the group consisting of A and T, X₁₃ is selected from N, V and K, X₁₄ is selected from the group consisting of R and L, and X₁₅ is selected from the group consisting of T, L and S |
| HCDR3 | DPHRTWWRYFDWLYPRGMDV (SEQ ID NO: 9) or GNFYYFDY (SEQ ID NO: 39) | LCDR3 | X₁₇QX₁₈X₁₉X₂₀X₂₁PX₂₂T (SEQ ID NO: 66), wherein X₁₇ is selected from the group consisting of Q and H, X₁₈ is selected from the group consisting of F and R, X₁₉ is selected from the group consisting of R and S, X₂₀ is selected from the group consisting of S and F, X₂₁ is selected from the group consisting of Y and S, and X₂₂ is selected from the group consisting of Y and L |

### Example 9. Preparation of Anti-FXI/FXIa Antibodies and Assay on the Binding Ability to Human FXI/FXIa

For the anti-FXI/FXIa murine or humanized antibody of the present disclosure, the heavy chain variable region was cloned into a mammalian cell expression vector containing a human antibody heavy chain IgG1, and the light chain variable region was cloned into a mammalian cell expression vector containing a constant region Cκ1 of a human antibody κ light chain; and ExpiCHO cells were transfected with expression vectors. Transfection was performed using ExpiCHO Expression System (Cat. no. A29133) at a ratio of 1 µg DNA/mL transfected cells according to the kit instructions. The cells were cultured by shaking in a shaker at 37 °C (8% CO₂) by standard methods. On day 8, the cell culture was collected and centrifuged at 4000 rpm, and the supernatant was collected and filtered through a 0.45 µm filter. The detection was performed, and the target antibody was obtained.

### Biacore assay

A certain amount of antibodies to be tested was affinity-captured using a Protein A biosensor chip (Cat. # 29127556, GE), then FXI/FXIa at a series of concentration gradients flowed through the surface of the chip. The reaction signals were detected in real time using a Biacore instrument (Biacore T200, GE) to obtain association and dissociation curves. After dissociation was completed for each cycle, the biochip was washed and regenerated with a regeneration solution prepared in a human antibody capture kit or a glycine-hydrochloric acid regeneration solution (pH 1.5, Cat. # BR-1003-54, GE). The buffer used in the experiment was HBS-EP + 10× buffer (Cat. # BR-1006-69, GE) diluted to 1× (pH 7.4) with double distilled water.

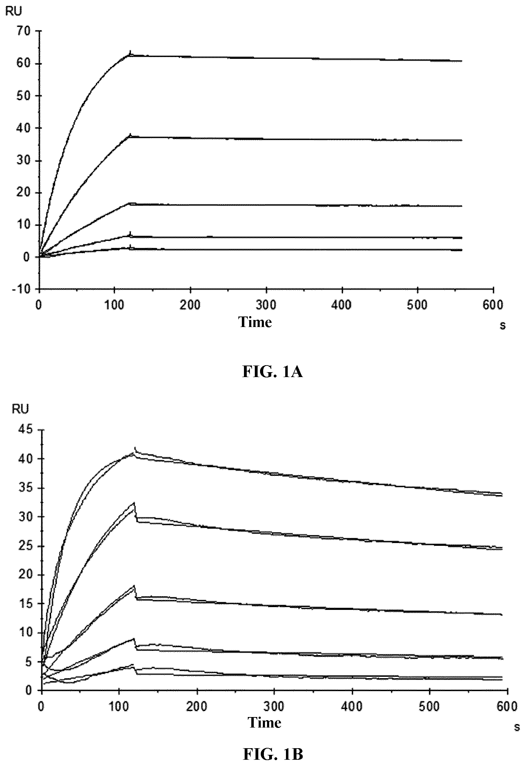

The data obtained from the experiment were fitted using BIAevaluation version 4.1, GE software using a (1: 1) Langmuir model to obtain affinity values. The test results of part of antibodies are shown in FIG. 1A, FIG. 1B and Table 6 below.

**Table 6. Affinity determination of anti-FXI/FXIa antibodies**

| Antibody | Antigen | ka(1/Ms) | kd(1/s) | K_{D}(M) |
|---|---|---|---|---|
| 3871 | FXI | 1.32E+06 | 2.66E-05 | 2.00E-11 |
| 3875 | FXI | 7.99E+05 | 3.02E-05 | 3.78E-11 |
| 3882 | FXI | 1.973E+6 | 5.846E-5 | 2.963E-11 |
| 0012 | FXIa | 5.43E+05 | 5.27E-04 | 1.06E-09 |
| 1209 | FXIa | 6.74E+05 | 4.73E-05 | 7.03E-11 |
| 1267 | FXIa | 8.73E+05 | 1.79E-05 | 2.22E-11 |
| 3807 | FXIa | 1.59E+06 | 2.35E-04 | 1.48E-10 |
| 3871 | FXIa | 1.24E+06 | 7.08E-04 | 5.70E-10 |
| 3875 | FXIa | 1.53E+06 | 3.36E-04 | 2.20E-10 |
| 3882 | FXIa | 1.06E+06 | 3.87E-04 | 3.64E-10 |

### Example 10. Assay on Inhibition of FXIa Enzyme Activity by Anti-FXI/FXIa Antibodies In Vitro

To test the function of the anti-FXI/FXIa antibodies, the ability of the anti-FXI/FXIa antibodies to bind to trypsin FXIa and inhibit the FXIa cleavage of a specific substrate, and the ability of the anti-FXI/FXIa antibodies to bind to trypsinogen FXI and block the FXIIa cleavage of FXI were assayed.

A SpectraMax M5 microplate reader was preset to 37 °C, a 384-well plate (thermo, Cat. 94410153) was precooled on ice, followed by the addition of 20 µL of the antibody to be tested (10 µg/mL) and 10 µL of FXIa trypsin (5 µg/mL), each diluted with 1× PBS. The plate was incubated at 37 °C for 5 min, then on ice for 5 min. After 10 µL of S-2366 (2 mM) (Chromogenix, S821090) was added, the kinetics curve at 405 nm was immediately read at 37 °C using the SpectraMax M5 microplate reader (once a minute, 60 min).

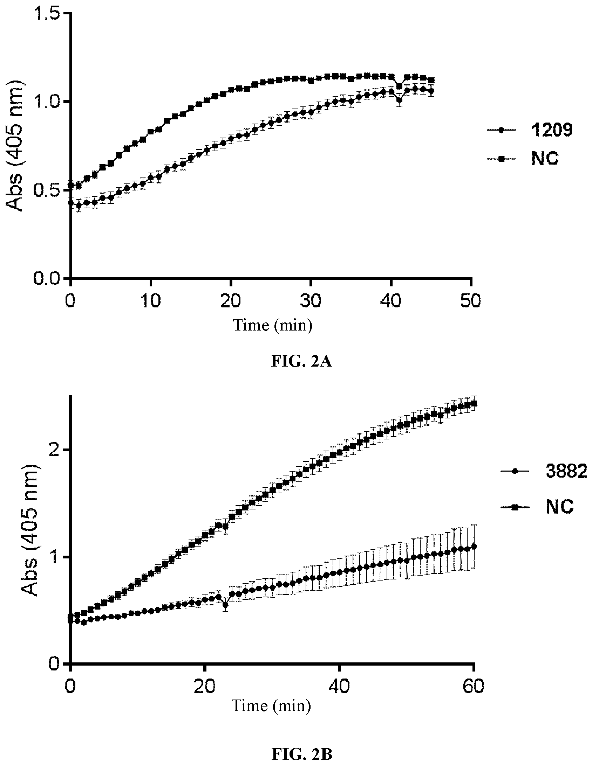

The test results of part of the antibodies are shown in FIG. 2A, wherein human IgG isotype was used as a negative control (NC).

The ability of the anti-FXI/FXIa antibodies to block the FXIIa cleavage of FXI was tested as follows: a 384-well plate was precooled on ice, followed by the addition of 10 µL of FXI (12 µg/mL), 10 µL of FXIIa (7.8 µg/mL), 10 µL of an FXI/FXIa antibody to be tested (300 µg/mL) and 10 µL of Dextran (100 µg/mL), each diluted with a buffer (20 mM HEPES, pH 7.4, 150 mM NaCl and 0.1% BSA); the plate was incubated at 37 °C for 60 min, then on ice for 5 min; after 10 µL of S-2366 (8 mM) (Chromogenix, S821090) was added, the plate was read using a microplate reader.

The test results of part of the antibodies are shown in FIG. 2B, wherein human IgG isotype was used as a negative control (NC).

### Example 11. Assay on aPTT and PT Anticoagulant Activity in Human/Monkey Blood

Human/monkey blood was collected freshly using sodium citrate tubes and centrifuged at 3000 rpm for 15 min, and the upper layer plasma was collected.

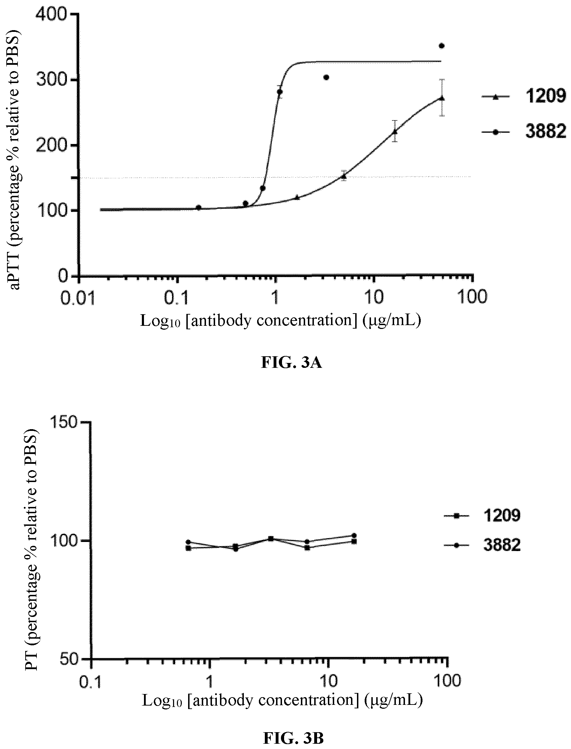

Activated partial thromboplastin time (aPTT) was determined with the Sysmex kit in the presence of different concentrations of the candidate antibody (diluted with PBS), and the candidate antibody to be tested was incubated with plasma at 37 °C for 3 min. Coagulation was then initiated by the addition of 25 mM calcium chloride reagent and the time when the coagulation occurred was determined. The concentration of candidate antibody that prolonged the aPTT by 50% relative to PBS (i.e., aPTT1.5) was determined. The rest results of part of the antibodies are shown in FIG. 3A, FIG. 4A and Table 7.

Prothrombin time (PT) was determined with the Sysmex kit in the presence of different concentrations of the candidate antibody (diluted with PBS), and the candidate antibody to be tested was incubated with plasma at 37 °C for 3 min. Coagulation was then initiated by the addition of thromboplastin and the time when the coagulation occurred was determined. The test results of part of the antibodies are shown in FIG. 3B and FIG. 4B.

The results showed that 3807, 3871, 3875 and 3882 reached 1.5-fold prolongation of aPTT at lower concentrations than Bay1213790, had a stronger inhibitory effect on FXI and inhibited the coagulation more effectively, and none of the above antibodies had the effect of prolonging PT.

**Table 7. Assay on aPTT1.5 of anti-FXI/FXIa antibodies**

| Antibody | Plasma | aPTT1.5(ug/mL^{∗}) |
|---|---|---|
| 0012 | Human blood | 14.59 |
| 1209 | Human blood | 4.68 |
| 3807 | Human blood | 1.33 |
| 3871 | Human blood | 0.88 |
| 3875 | Human blood | 0.87 |
| 3882 | Human blood | 0.63 |
| Bay1213790 | Human blood | 2.15 |
| 0012 | Monkey blood | >8.33 |
| 1209 | Monkey blood | 4.08 |
| 3807 | Monkey blood | 1.00 |
| 3871 | Monkey blood | 1.12 |
| 3882 | Monkey blood | 1.14 |
| Bay123790 | Monkey blood | 2.73 |

| | | |
|---|---|---|
| ^{∗}The concentration is the final concentration of the system. | | |

### Example 12. Pharmacokinetic (PK)/Pharmacodynamic (PD) Assay in Cynomolgus Monkeys

Normal adult male cynomolgus monkeys (body weight in the range of 4.0-4.7 kg) were grouped according to body weight (monkeys weighing 1st, 4th and 5th were assigned to one group, and monkeys weighing 2nd, 3rd and 6th were assigned to the other group), 3 animals in each group, and observed for 14 days.

Blood samples were collected one day before administration using sodium citrate blood collection tubes, and as the samples before administration, were determined for aPTT, PT, plasma drug concentration, plasma active XI factor (Factor XI, FXI) ratio (FXI:C %, i.e., the proportion of FXI with coagulation activity), and plasma free FXI concentration; and bleeding time was also determined for each animal. Of the two groups of animals, one was blank and not administered; and the other was administered with 3882, 5 mg/kg body weight (mg/kg, mpk). The compound was dissolved in phosphate buffered saline (PBS) at a concentration of 5 mg/mL and administered by intravenous injection.

The blank control group was observed for bleeding time 15 minutes (min) and 3 hours (h) after the administration; and plasma was collected and observed for aPTT and PT 15 min, 3 h, 6 h and 1 day (d) after the administration according to the above method described above. The administration group was observed for bleeding time 15 min, 3 h, 2 d, 4 d, 1 week (w), 2 w and 3 w after the administration; and plasma was collected and observed for aPTT, PT, plasma drug concentration, plasma FXI:C % and plasma free FXI concentration 15 min, 3 h, 6 h, 1 d, 2 d, 4 d, 1 w, 2 w, 3 w, 4 w, 5 w and 6 w after the administration according to the method described above. AV-shunt thrombus construction was performed on both groups of animals 1 d after the administration, wherein the thrombus construction time was 10 min, and the net weight of the thrombus was weighed after thrombus construction.

aPTT, PT and plasma FXI:C % were determined by a coagulometer and corresponding kits, and plasma free FXI concentration and plasma drug concentration are determined by an ELISA method. The thrombus weight of the administration group was compared with that of the control group, and statistically analyzed by t-test.

The test results of the antibodies are shown in FIGs. 5A-5D, in which the negative control (NC) was not administered. The result showed that 5 mpk well inhibited thrombogenesis and prolonged the endogenous coagulation time, but had no significant influence on the bleeding time and the exogenous coagulation time of animals, and the PK result showed that the half-life period was about 20 days. "^{∗∗}" in FIG. 5B represents P value < 0.01, which indicates a significant difference.

### Example 13. Pharmacodynamic (PD) Assay in Cynomolgus Monkeys

6 normal adult male cynomolgus monkeys (body weight in the range of 7-9 kg) were randomly divided into 3 groups, which were:
BAY1213790 intravenous administration 1 mg/kg body weight (mg/kg) group;
3882 intravenous administration 1 mg/kg body weight (mg/kg) group;
3882 subcutaneous administration 1 mg/kg body weight (mg/kg) group.

The blood samples of animals in the intravenous administration group were collected before the administration and 5 min, 1 h, 1 d, 2 d, 3 d, 5 d, 1 w, 2 w, 3 w and 4 w after the administration, the blood samples of animals in the subcutaneous administration group were collected before the administration and 1 h, 1 d, 2 d, 3 d, 5 d, 1 w, 2 w, 3 w and 4 w after the administration, both with sodium citrate blood collection tubes, and aPTT and plasma FXI:C % were determined with a coagulometer and corresponding kits.

The test results are shown in FIG. 6A and FIG. 6B. The results showed that 3882 was able to significantly prolong the endogenous coagulation time and inhibit the activity of FXI by both subcutaneous administration and intravenous administration. 3882 has longer maintenance time for prolonging the endogenous coagulation time and stronger inhibition effect on FXI compared with BAY1213790 when administered at an equal dose.
> Heavy chain of BAY1213790: 
EVQLLESGGGLVQPGGSLRLSCAASGFTFSQYGMDWVRQAPGKGLEWVSGIGP
SGGSTVYADSVKGRFTISRDNSKNTLYLQMNSLRAEDTAVYYCTRGGPYYYYG
MDVWGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPG (SEQ ID NO: 68)
> Light chain of BAY1213790:

DIQMTQSPSSLSASVGDRVTITCQASQDISNYLNWYQQKPGKAPKLLIYDASNL
ETGVPSRFSGSGSGTDFTFTISSLQPEDIATYYCQQADSFPVTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
(SEQ ID NO: 69)

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. An anti-FXI/FXIa antibody or an antigen-binding fragment thereof, comprising:
a heavy chain HCDR1 comprising a sequence set forth in SEQ ID NO: 63;
a heavy chain HCDR2 comprising a sequence set forth in SEQ ID NO: 64;
a heavy chain HCDR3 comprising a sequence set forth in SEQ ID NO: 9 or SEQ ID NO: 39;
a light chain LCDR1 comprising a sequence set forth in SEQ ID NO: 10 or SEQ ID NO: 40;
a light chain LCDR2 comprising a sequence set forth in SEQ ID NO: 65; and
a light chain LCDR3 comprising a sequence set forth in SEQ ID NO: 66.

2. The anti-FXI/FXIa antibody or the antigen-binding fragment thereof according to claim 1, comprising:
(a) heavy chain HCDR1, HCDR2 and HCDR3 comprising sequences set forth in SEQ ID NOs: 37, 38 and 39, respectively, and light chain LCDR1, LCDR2 and LCDR3 comprising sequences set forth in SEQ ID NOs: 40, 41 and 42, respectively;
(b) heavy chain HCDR1, HCDR2 and HCDR3 comprising sequences set forth in SEQ ID NOs: 7, 8 and 9, respectively, and light chain LCDR1, LCDR2 and LCDR3 comprising sequences set forth in SEQ ID NOs: 10, 11 and 12, respectively;
(c) heavy chain HCDR1, HCDR2 and HCDR3 comprising sequences set forth in SEQ ID NOs: 22, 23 and 9, respectively, and light chain LCDR1, LCDR2 and LCDR3 comprising sequences set forth in SEQ ID NOs: 10, 29 and 12, respectively;
(d) heavy chain HCDR1, HCDR2 and HCDR3 comprising sequences set forth in SEQ ID NOs: 24, 25 and 9, respectively, and light chain LCDR1, LCDR2 and LCDR3 comprising sequences set forth in SEQ ID NOs: 10, 29 and 12, respectively;
(e) heavy chain HCDR1, HCDR2 and HCDR3 comprising sequences set forth in SEQ ID NOs: 26, 27 and 9, respectively, and light chain LCDR1, LCDR2 and LCDR3 comprising sequences set forth in SEQ ID NOs: 10, 29 and 12, respectively; or
(f) heavy chain HCDR1, HCDR2 and HCDR3 comprising sequences set forth in SEQ ID NOs: 28, 25 and 9, respectively, and light chain LCDR1, LCDR2 and LCDR3 comprising sequences set forth in SEQ ID NOs: 10, 29 and 12, respectively.

3. The anti-FXI/FXIa antibody or the antigen-binding fragment thereof according to any one of claims 1 to 2, being a murine antibody, a chimeric antibody, a humanized antibody or a human antibody, or a fragment thereof, wherein
preferably, the anti-FXI/FXIa antibody or the antigen-binding fragment thereof is a humanized antibody or a fragment thereof; and
more preferably, the humanized antibody or the fragment thereof has a light chain template of IGKV3-11^{∗}01 and a heavy chain template of IGHV1-69-2*01.

4. The anti-FXI/FXIa antibody or the antigen-binding fragment thereof according to claim 3, comprising a VH having a back mutation of any one or any combination of Y27F, T28N, F29I, T30K, A93L, R94Y, E73T, R66K, V67A, T75A and T76N, and/or a VL having a back mutation of any one or any combination of R45K, L46R, L47W, I58V and F71Y

5. The anti-FXI/FXIa antibody or the antigen-binding fragment thereof according to any one of claims 1 to 4, comprising:
a VH set forth in one of SEQ ID NOs: 5, 17-20, 30-33, 35, 43, 45-49 and 53-58 or a VH having at least 90% identity thereto, and
a VL set forth in one of SEQ ID NOs: 6, 21, 34, 36, 44 and 50-52 or a VL having at least 90% identity thereto.

6. The anti-FXI/FXIa antibody or the antigen-binding fragment thereof according to claim 5, comprising:
a VH set forth in SEQ ID NO: 58 or a VH having at least 90% identity thereto, and
a VL set forth in SEQ ID NO: 51 or a VL having at least 90% identity thereto;
a VH set forth in SEQ ID NO: 5 or a VH having at least 90% identity thereto, and
a VL set forth in SEQ ID NO: 6 or a VL having at least 90% identity thereto;
a VH set forth in SEQ ID NO: 17 or a VH having at least 90% identity thereto, and
a VL set forth in SEQ ID NO: 21 or a VL having at least 90% identity thereto;
a VH set forth in SEQ ID NO: 18 or a VH having at least 90% identity thereto, and
a VL set forth in SEQ ID NO: 21 or a VL having at least 90% identity thereto;
a VH set forth in SEQ ID NO: 19 or a VH having at least 90% identity thereto, and
a VL set forth in SEQ ID NO: 21 or a VL having at least 90% identity thereto;
a VH set forth in SEQ ID NO: 20 or a VH having at least 90% identity thereto, and
a VL set forth in SEQ ID NO: 21 or a VL having at least 90% identity thereto;
a VH set forth in SEQ ID NO: 30 or a VH having at least 90% identity thereto, and
a VL set forth in SEQ ID NO: 34 or a VL having at least 90% identity thereto;
a VH set forth in SEQ ID NO: 31 or a VH having at least 90% identity thereto, and
a VL set forth in SEQ ID NO: 34 or a VL having at least 90% identity thereto;
a VH set forth in SEQ ID NO: 32 or a VH having at least 90% identity thereto, and
a VL set forth in SEQ ID NO: 34 or a VL having at least 90% identity thereto;
a VH set forth in SEQ ID NO: 35 or a VH having at least 90% identity thereto, and
a VL set forth in SEQ ID NO: 36 or a VL having at least 90% identity thereto;
a VH set forth in SEQ ID NO: 43 or a VH having at least 90% identity thereto, and
a VL set forth in SEQ ID NO: 44 or a VL having at least 90% identity thereto;
a VH set forth in SEQ ID NO: 45 or a VH having at least 90% identity thereto, and
a VL set forth in SEQ ID NO: 51 or a VL having at least 90% identity thereto;
a VH set forth in SEQ ID NO: 49 or a VH having at least 90% identity thereto, and
a VL set forth in SEQ ID NO: 51 or a VL having at least 90% identity thereto.

7. The anti-FXI/FXIa antibody or the antigen-binding fragment thereof according to any one of claims 1 to 6, having a VH linked to human or mouse CH1 and a VL linked to human or mouse CL or Cκ, wherein
preferably, the human CH1 is set forth in SEQ ID NO: 13 or 59, and the Cκ has a sequence set forth in SEQ ID NO: 14.

8. The anti-FXI/FXIa antibody or the antigen-binding fragment thereof according to any one of claims 1 to 7, comprising a constant domain Fc, wherein
preferably, the Fc is an Fc of IgG1, an Fc of IgG4, or an Fc of IgG4P; and
more preferably, the Fc of IgG1 has a sequence set forth in SEQ ID NO: 67, and the Fc of IgG4P has a sequence set forth in SEQ ID NO: 60.

9. The anti-FXI/FXIa antibody or the antigen-binding fragment thereof according to any one of claims 1 to 8, comprising:
a heavy chain set forth in SEQ ID NO: 61 or a heavy chain having at least 80% identity thereto, and
a light chain set forth in SEQ ID NO: 62 or a light chain having at least 80% identity thereto; or
a heavy chain set forth in SEQ ID NO: 15 or a heavy chain having at least 80% identity thereto, and
a light chain set forth in SEQ ID NO: 16 or a light chain having at least 80% identity thereto.

10. The anti-FXI/FXIa antibody or the antigen-binding fragment thereof according to any one of claims 1 to 9, wherein the antigen-binding fragment is selected from the group consisting of an scFv fragment, an Fv fragment, a Fab fragment and a Fab' fragment.

11. The anti-FXI/FXIa antibody or the antigen-binding fragment thereof according to any one of claims 1 to 10, having any one or more of the following properties:
preventing or arresting activation of a coagulation pathway;
blocking binding of FXI/FXIa to one or more of FIX, FXIIa and thrombin;
blocking binding of one or more of FIX, FXI and FXIa to a platelet receptor;
binding to a human FXI and/or FXIa protein with an affinity of less than or equal to 10⁻⁹ K_{D};
preventing the FXI/FXIa catalytic domain from presenting an active conformation when binding to FXI/FXIa; and
having the ability to be used for subcutaneous administration or intravenous administration.

12. A polynucleotide encoding the anti-FXI/FXIa antibody or the antigen-binding fragment thereof according to any one of claims 1 to 11.

13. A vector comprising the polynucleotide according to claim 12.

14. A host cell comprising the vector according to claim 13.

15. A method for preparing an anti-FXI/FXIa antibody or an antigen-binding fragment thereof, comprising:
expressing the anti-FXI/FXIa antibody or the antigen-binding fragment thereof in the host cell according to claim 14, and
isolating the anti-FXI/FXIa antibody or the antigen-binding fragment thereof from the host cell.

16. A pharmaceutical composition, comprising:
- a pharmaceutically acceptable excipient, diluent or carrier; and
- any one or any combination selected from the group consisting of the following: the anti-FXI/FXIa antibody or the antigen-binding fragment thereof according to any one of claims 1 to 11, the polynucleotide according to claim 12, and the vector according to claim 13; wherein
preferably, the pharmaceutical composition is a subcutaneous injection or an intravenous injection.

17. Use of any one or any combination selected from the group consisting of the following in the preparation of a medicament or a pharmaceutical composition:
the anti-FXI/FXIa antibody or the antigen-binding fragment thereof according to any one of claims 1 to 11, the polynucleotide according to claim 12, and the vector according to claim 13;
wherein the medicament or pharmaceutical composition is used for treating and/or preventing any one selected from the group consisting of the following: a thrombotic or thromboembolic disorder, a thrombotic or thromboembolic complication, cardiac arrhythmia, cardiogenic thromboembolism, and disseminated intravascular coagulation;
preferably, the thrombotic or thromboembolic disorder or the complication thereof is selected from the group consisting of: cardiac coronary artery disease, ST-elevation myocardial infarction (STEMI), non-ST-elevation myocardial infarction (non-STEMI), stable angina pectoris, unstable angina pectoris, reocclusion and restenosis after coronary interventions, peripheral arterial occlusive disease, pulmonary embolism, venous thromboembolism, venous thrombosis, transient ischemic attack, thrombotic stroke and thromboembolic stroke, pulmonary disease caused by chronic thromboembolic pulmonary hypertension (CTEPH), pulmonary hypertension caused by CTEPH, and a combination thereof;
preferably, the cardiac coronary artery disease is acute coronary syndrome.

18. A method for treating and/or preventing a disease, wherein the disease is selected from any one of the following:
a thrombotic or thromboembolic disorder, a thrombotic or thromboembolic complication, cardiac arrhythmia, cardiogenic thromboembolism, and disseminated intravascular coagulation;
the method comprises the step of:
administering to a subject the anti-FXI/FXIa antibody or the antigen-binding fragment thereof according to any one of claims 1 to 11, the polynucleotide according to claim 12, the vector according to claim 13, or any combination thereof in an effective amount for treating or preventing the disease;
preferably, the thrombotic or thromboembolic disorder or the complication thereof is selected from the group consisting of: cardiac coronary artery disease, ST-elevation myocardial infarction (STEMI), non-ST-elevation myocardial infarction (non-STEMI),
stable angina pectoris, unstable angina pectoris, reocclusion and restenosis after coronary interventions, peripheral arterial occlusive disease, pulmonary embolism,
venous thromboembolism, venous thrombosis, transient ischemic attack, thrombotic stroke and thromboembolic stroke, pulmonary disease caused by chronic thromboembolic pulmonary hypertension (CTEPH), pulmonary hypertension caused by CTEPH, and a combination thereof;
preferably, the cardiac coronary artery disease is acute coronary syndrome;
preferably, the subject has ischemic stroke and/or deep vein thrombosis related to atrial fibrillation.
